# EUROPEAN PATENT APPLICATION

(11) **EP 2 045 250 A1**
(43) Date of publication of application: **08.04.2009**
(21) Application number: 07117266.2
(22) Date of filing: 26.09.2007
(51) Int. Cl.: C07D 401/06, C07D 403/06, A61K 31/454, A61K 31/4025, A61P 29/00

(54) **Ring-substituted phenyl pyrrole aminoguanidine derivatives**

(71) Applicant: Action Pharma A/S, 8200 Arhus N (DK)
(72) Inventor: Jonassen, Thomas Engelbrecht Norkild, 2840, Holte (DK)
(74) Representative: Plougmann & Vingtoft A/S

(57) **Abstract**

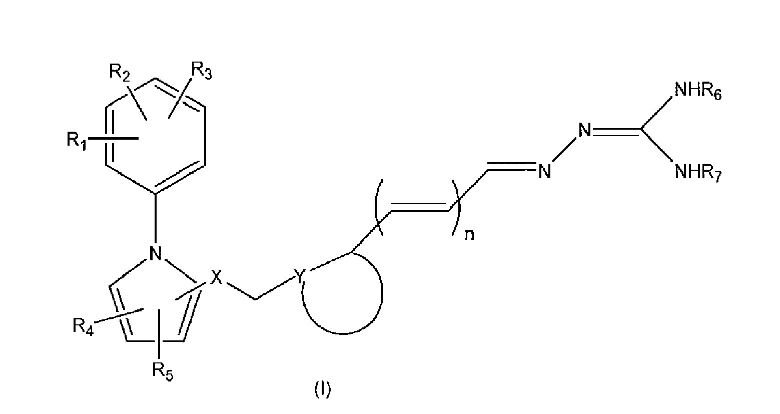

The present invention relates to novel compounds of the general formula (I). The present invention further relates to the use of such compounds or pharmaceutically acceptable salts thereof for the treatment of diseases associated with the melanocortin receptors or related systems.

## Description

### FIELD OF THE INVENTION

The present invention relates to phenyl pyrrole aminoguanidine derivatives. The present invention further relates to the use of such phenyl pyrrole aminoguanidine derivatives for the treatment of diseases associated with the melanocortin receptors or related systems, e.g. the melanocyte stimulating hormones.

### BACKGROUND OF THE INVENTION

A number of large linear and cyclic peptides are known in the art which show high specific binding to melanocortin (MC) receptors. The agonistic and/or antagonistic properties of these peptides are also known. See, for example, WO 99/21571.

Moreover, a number of low molecular weight compounds are known, e.g., isoquinolines, spiropyridines and benzimidazoles, which show activity on the MC receptors. See, for example, WO 99/55679, WO 99/64002 and WO 01/05401. For further literature disclosing other compounds also acting on the MC receptors, reference is made to WO 00/74679, WO 00/58361, WO 02/18327, WO 02/12166, WO 01/55106, WO 01/55107, WO 01/55109, WO 02/11715 and WO 02/12178.

However, there is still a large need to provide low molecular weight compounds showing agonistic or antagonistic properties to the MC receptors. The compounds of the present invention are structurally different from the above-mentioned compounds and, consequently, constitute a new class of compounds that show activity to the MC receptors.

Prior art compounds, which have some structural relationship to the compounds of the present invention include the compounds described in W0 98/23267: 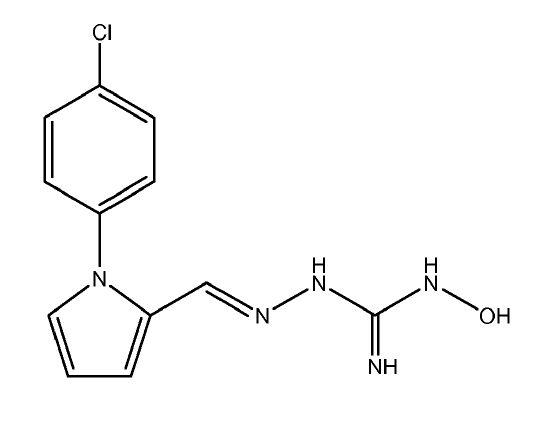 This hydroxyguanidine derivative has proven activity against xanthine oxidase/xanthine dehydrogenase enzymes.

Furthermore, compounds disclosed in WO 03/013509 also posses a structure similar to the compounds of the invention. The general structure of the compounds disclosed in WO 03/013509 is as follows: 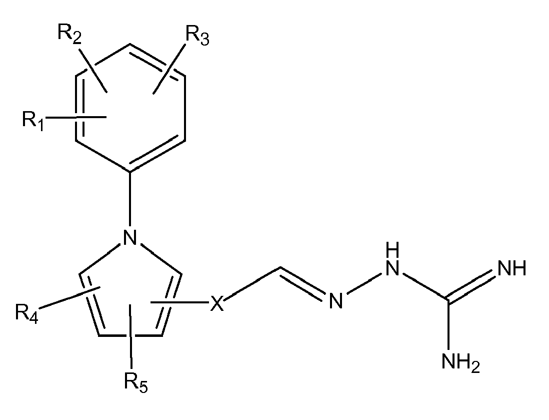 where X is (CH₂)ₙ, and n is 0, 1 or 2.

The compounds of WO 03/013509 exhibit antiinflammatory properties and a capability to stimulate melanocortin receptors (MC receptors). Though these compounds are able to stimulate MC receptors, they do not posses a high binding affinity to the MC-receptors compared to the native agonist, α-MSH. The binding affinity of the compounds of WO 03/013509 for the MC-receptors lies within the sub-micromolar to micromolar range compared to α-MSH, which possesses a binding affinity for the MC-receptors lying in the nanomolar range. Furthermore, the compounds of WO 03/013509 are light-sensitive, i.e. light can induce a conversion from the trans isomeric form of the compounds to the cis isomeric form of the compounds. Trans-Cis conversion is well-known to be induced by light.

### SUMMARY OF THE INVENTION

The compounds of the present invention differ from the compounds disclosed in WO 03/013509 in that the aminoguanidine substituent of the pyrrole ring has been modified in such a way that that the conjugated double bonds of the pyrrole ring and the conjugated double bonds of the aminoguanidine substituent are separated by a ring system. This, in turn, has the consequence that the compounds of the invention posses a higher binding affinity for the MC-receptors compared to the compounds disclosed in WO 03/013509.

Accordingly, one object of the present invention is to provide compounds which exhibit an increased binding affinity for one or more of the MC-1, MC-3, MC-4 and M-5 receptor compared to the compounds described in WO 03/013509. Another object of the present invention is to provide compounds which, compared to the compounds disclosed in WO 03/013509, are less light-sensitive (i.e. which are more stable).

Thus, in a first aspect the present invention relates to a compound of the general formula (I) 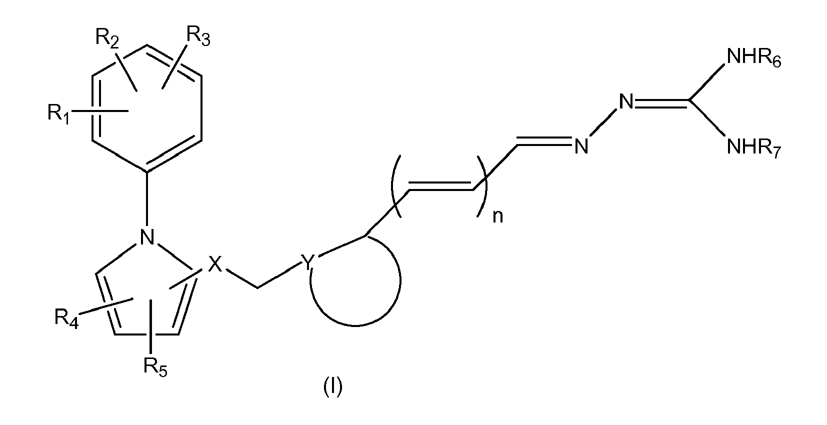 including tautomeric and isomeric forms, such as enantiomeric forms, diastereomeric forms and racemic forms, thereof,
wherein
X is (CH₂)ₘ, and m is 0, 1 or 2;
n is 0, 1 or 2;
Y is nitrogen (N) or carbon (C);
∪ represents, together with Y and the carbon atom covalently linked to Y, an optionally substituted five- or six-membered heterocyclyl or cycloalkyl group; each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclyl amino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine; each R₆ and R₇ is independently selected from the group consisting of hydrogen, optionally substituted C₁₋₆-alkyl, optionally substituted C₂₋₆-alkenyl, optionally substituted C₄₋₆-alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino-C₁₋₆-alkyl-aminocarbonyl and mono- and di(C₁₋₆-alkyl)amino-C₁₋₆-alkyl-aminocarbonyl; or R₆ and R₇ may together form a five- or six-membered nitrogen-containing ring; or a pharmaceutically acceptable salt thereof.

In a further aspect the present invention relates to a pharmaceutical composition comprising a compound of the invention and a pharmaceutically acceptable carrier or excipient.

In a still further aspect the present invention relates to a dosage form comprising a pharmaceutical composition of the invention.

In yet another aspect the present invention relates to a compound of the invention for use a medicament.

In an even further aspect the present invention relates to the use of a compound of the invention for the manufacture of a medicament for the treatment of a disease selected from the group consisting of inflammatory conditions, e.g. acute or chronic inflammatory conditions, diabetes mellitus, insulin-resistance, sexual dysfunction including dysfunction of male erection, eating disorders including anorexia, obesity, mental disorders, dysfunction of the endocrine system, drug-induced disorders of the blood and lymphoid system, allergy disorders, disorders of the cardiovascular system and pain.

Analogously, the present invention also relates to a method of treating a mammal having a disease or disorder selected from the group consisting of inflammatory conditions, e.g. acute or chronic inflammatory conditions, diabetes mellitus, insulin-resistance, sexual dysfunction including dysfunction of male erection, eating disorders including anorexia, obesity, mental disorders, dysfunction of the endocrine system, drug-induced disorders of the blood and lymphoid system, allergy disorders, disorders of the cardiovascular system and pain, said method comprising administering to said mammal a therapeutically effective amount of a compound of the invention.

Other aspects of the present invention will be apparent from the appended claims and the below description.

### BRIEF DESCRIPTION OF THE DRAWINGS

Fig. 1 shows a possible synthetic route to the compounds of the invention, wherein Y is nitrogen (N).
Fig. 2 shows a possible synthetic route to the compounds of the invention, wherein Y is carbon (C).

Fig. 3 shows specific phenyl pyrrole aminoguanidine derivatives of the invention.

### DETAILED DESCRIPTION OF THE INVENTION

### Definitions

In the present context, the term "C₁₋₆-alkyl" is intended to mean a linear or branched hydrocarbon group having 1 to 6 carbon atoms, such as methyl, ethyl, *n*-propyl, *iso-*propyl, *n*-butyl, iso-butyl, *sec*-butyl, tert-butyl, *n*-pentyl, iso-pentyl, *neo*-pentyl and n-hexyl, and the term "C₁₋₄-alkyl" is intended to cover a linear or branched hydrocarbon group having 1 to 4 carbon atoms, e.g. methyl, ethyl, *n*-propyl, iso-propyl, *n*-butyl, *iso-*butyl, *sec*-butyl and tert-butyl.

Whenever the term "C₁₋₆-alkyl" is used herein, it should be understood that a particularly interesting embodiment thereof is "C₁₋₄-alkyl".

When used herein, the term "C₃₋₆-cycloalkyl" is intended to mean a cyclic hydrocarbon group having 3 to 6 carbon atoms, such as cyclopropyl, cyclobutyl, cyclopentyl and cyclohexyl. Similarly, the term "five- or six-membered cycloalkyl group" refers to cyclopentyl and cyclohexyl.

Herein, the terms "C₂₋₆-alkenyl" and "C₄₋₆-alkadienyl", are intended to cover linear or branched hydrocarbon groups having 2 to 6 and 4 to 6, carbon atoms, respectively, and comprising one and two unsaturated bonds, respectively. Examples of alkenyl groups are vinyl, allyl, butenyl, pentenyl and hexenyl. Examples of alkadienyl groups include butadienyl, pentadienyl and hexadienyl. Preferred examples of alkenyl are vinyl, allyl and butenyl, especially allyl.

In the present context the term "C₂₋₆-alkynyl" is intended to mean a linear or branched hydrocarbon group having 2 to 6 carbon atoms and containing one or more triple bonds. Illustrative examples of C₂₋₆-alkynyl groups include acetylene, propynyl, butynyl, as well as branched forms of these. The position of unsaturation (the triple bond) may be at any position along the carbon chain. More than one bond may be unsaturated such that the "C₂₋₆-alkynyl" is a di-yne or enedi-yne as is known to the person skilled in the art.

When used herein the term "C₁₋₆-alkoxy" is intended to mean C₁₋₆-alkyl-oxy, such as methoxy, ethoxy, *n*-propoxy, iso-propoxy, n-butoxy, iso-butoxy, sec-butoxy, tert-butoxy, *n*-pentoxy, iso-pentoxy, *neo*-pentoxy and n-hexoxy, and the term "C₁₋₄-alkoxy" is intended to mean C₁₋₄-alkyl-oxy, e.g. methoxy, ethoxy, *n*-propoxy, iso-propoxy, n-butoxy, *iso-*butoxy, sec-butoxy and tert-butoxy.

Whenever the term "C₁₋₆-alkoxy" is used herein, it should be understood that a particularly interesting embodiment thereof is "C₁₋₄-alkoxy".

Likewise, the term "C₂₋₆-alkenyl-oxy" is intended to mean C₂₋₆-alkenyl-oxy.

Herein, the term "halogen" includes fluoro, chloro, bromo, and iodo. In particular, fluoro, chloro and bromo are preferred.

In the present context, i.e. in connection with the terms "alkyl", "five- or six-membered cycloalkyl group" "alkenyl", "alkadienyl" and "alkynyl", the term "optionally substituted" is intended to mean that the group in question may be substituted one or several times, preferably 1-3 times, with group(s) selected from hydroxy (which when bound to an unsaturated carbon atom may be present in the tautomeric keto form), C₁₋₆-alkoxy, C₂₋₆-alkenyloxy, carboxy, oxo (forming a keto or aldehyde functionality), C₁₋₆-alkoxycarbonyl, C₁₋₆-alkylcarbonyl, formyl, aryl, aryloxycarbonyl, aryloxy, arylamino, arylcarbonyl, heteroaryl, heteroarylamino, heteroaryloxycarbonyl, heteroaryloxy, heteroarylcarbonyl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, cyano, guanidino, carbamido, C₁₋₆-alkyl-sulphonyl-amino, aryl-sulphonyl-amino, heteroaryl-sulphonyl-amino, C₁₋₆-alkanoyloxy, C₁₋₆-alkyl-sulphonyl, C₁₋₆-alkyl-sulphinyl, C₁₋₆-alkylsulphonyloxy, nitro, C₁₋₆-alkylthio and halogen, where any aryl and heteroaryl may be substituted as specifically describe below for "optionally substituted aryl and heteroaryl", and any alkyl, alkoxy, and the like representing substituents may be substituted with hydroxy, C₁₋₆alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine.

Preferably, the above-mentioned substituents are selected from hydroxy (which when bound to an unsaturated carbon atom may be present in the tautomeric keto form), C₁₋₆-alkoxy (i.e. C₁₋₆-alkyl-oxy), C₂₋₆-alkenyloxy, carboxy, oxo (forming a keto or aldehyde functionality), C₁₋₆-alkylcarbonyl, formyl, aryl, aryloxy, arylamino, arylcarbonyl, heteroaryl, heteroarylamino, heteroaryloxy, heteroarylcarbonyl, amino, mono- and di(C₁₋₆-alkyl)amino; carbamoyl, mono- and di(C₁₋₆-alkyl)aminocarbonyl, amino-C₁₋₆-alkylaminocarbonyl, mono- and di(C₁₋₆-alkyl)amino-C₁₋₆-alkyl-aminocarbonyl, C₁₋₆-alkylcarbonylamino, guanidino, carbamido, C₁₋₆-alkyl-sulphonyl-amino, C₁₋₆-alkyl-sulphonyl, C₁₋₆-alkylsulphinyl, C₁₋₆-alkylthio and halogen, where any aryl and heteroaryl may be substituted as specifically describe below for "optionally substituted aryl and heteroaryl".

Especially preferred examples of such substituents are hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino and guanidine, in particular halogen. Thus, particularly preferred "optionally substituted C₁₋₆-alkyl" groups include halogen-substituted alkyl groups, such as trihalo-C₁₋₆-alkyl, such as tribromomethyl, trichloromethyl or trifluoromethyl.

The term "optionally substituted C₁₋₆-alkoxy" is intended to mean that the alkoxy groups may be substituted one or several times, preferably 1-3 times, with group(s) selected from hydroxy (which when bound to an unsaturated carbon atom may be present in the tautomeric keto form), C₁₋₆-alkoxy (i.e. C₁₋₆-alkyl-oxy), C₂₋₆-alkenyloxy, carboxy, oxo (forming a keto or aldehyde functionality), C₁₋₆-alkoxycarbonyl, C₁₋₆-alkylcarbonyl, formyl, aryl, aryloxycarbonyl, aryloxy, arylcarbonyl, heteroaryl, heteroaryloxycarbonyl, heteroaryloxy, heteroarylcarbonyl, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, cyano, guanidino, carbamido, C₁₋₆-alkyl-sulphonyl-amino, aryl-sulphonyl-amino, heteroaryl-sulphonyl-amino, C₁₋₆-alkanoyloxy, C₁₋₆-alkyl-sulphonyl, C₁₋₆-alkyl-sulphinyl, C₁₋₆-alkylsulphonyloxy, nitro, C₁₋₆-alkylthio and halogen, where any aryl and heteroaryl may be substituted as specifically describe below for "optionally substituted aryl and heteroaryl".

Especially preferred examples of such substituents are and those carrying one or two substituents selected from hydroxy, C₁₋₆-alkyl, C₁₋₆-alkoxy, C₂₋₆-alkenyloxy, carboxy, halogen or C₁₋₆-alkylthio.

In the present context the term "aryl" is intended to mean a fully or partially aromatic carbocyclic ring or ring system, such as phenyl, naphthyl, 1,2,3,4-tetrahydronaphthyl, anthracyl, phenanthracyl, pyrenyl, benzopyrenyl, fluorenyl and xanthenyl, among which phenyl is a preferred example.

The term "heteroaryl" is intended to mean a fully or partially aromatic carbocyclic ring or ring system where one or more of the carbon atoms have been replaced with heteroatoms, e.g. nitrogen (=N- or -NH-), sulphur, and/or oxygen atoms. Examples of such heteroaryl groups are oxazolyl, isoxazolyl, thiazolyl, isothiazolyl, pyrrolyl, imidazolyl, pyrazolyl, pyridinyl, pyrimidinyl, pyrazinyl, pyridazinyl, triazinyl, coumaryl, furyl, thienyl, quinolyl, benzothiazolyl, benzotriazolyl, benzodiazolyl, benzooxozolyl, phthalazinyl, phthalanyl, triazolyl, tetrazolyl, isoquinolyl, acridinyl, carbazolyl, dibenzazepinyl, indolyl, benzopyrazolyl and phenoxazonyl.

Particularly interesting heteroaryl groups are oxazolyl, isoxazolyl, thiazolyl, isothiazolyl, pyrrolyl, imidazolyl, pyrazolyl, pyridinyl, pyrimidinyl, pyrazinyl, pyridazinyl, furyl, thienyl, quinolyl, triazolyl, tetrazolyl, isoquinolyl and indolyl, in particular pyrrolyl, imidazolyl, pyridinyl, pyrimidinyl, thienyl, quinolyl, tetrazolyl and isoquinolyl.

In the present context, the term "heterocyclyl" is intended to mean a non-aromatic carbocyclic ring or ring system where one or more of the carbon atoms have been replaced with heteroatoms, e.g. nitrogen (=N- or -NH-), sulphur, and/or oxygen atoms. Examples of such heterocyclyl groups are imidazolidine, piperazine, hexahydropyridazine, hexahydropyrimidine, diazepane, diazocane, pyrrolidine, piperidine, azepane, azocane, aziridine, azirine, azetidine, pyroline, tropane, oxazinane (morpholine), azepine, dihydroazepine, tetrahydroazepine, hexahydroazepine, oxazolane, oxazepane, oxazocane, thiazolane, thiazinane, thiazepane, thiazocane, oxazetane, diazetane, thiazetane, tetrahydrofuran, tetrahydropyran, oxepane, tetrahydrothiophene, tetrahydrothiopyrane, thiepane, dithiane, dithiepane, dioxane, dioxepane, oxathiane and oxathiepane.

Preferred examples of heterocyclyl groups are imidazolidine, piperazine, hexahydropyridazine, hexahydropyrimidine, diazepane, diazocane, pyrrolidine, piperidine, azepane, azocane, azetidine, tropane, oxazinane (morpholine), oxazolane, oxazepane, thiazolane, thiazinane, and thiazepane, in particular imidazolidine, piperazine, hexahydropyridazine, hexahydropyrimidine, diazepane, pyrrolidine, piperidine, azepane, oxazinane (morpholine) and thiazinane. An example of a particularly preferred five-membered heterocyclyl group is pyrrolidine, and an example of a particularly preferred six-membered heterocyclyl group is piperidine.

In the present context, i.e. in connection with the terms "aryl", "heteroaryl", and "heterocyclyl", the term "optionally substituted" is intended to mean that the group in question may be substituted one or several times, preferably 1-5 times, in particular 1-3 times, with group(s) selected from hydroxy (which when present in an enol system may be represented in the tautomeric keto form), C₁₋₆-alkyl, C₁₋₆-alkoxy, C₂₋₆-alkenyloxy, oxo (which may be represented in the tautomeric enol form), carboxy, C₁₋₆-alkoxycarbonyl, C₁₋₆-alkylcarbonyl, formyl, aryl, aryloxy, arylamino, aryloxycarbonyl, arylcarbonyl, heteroaryl, heteroarylamino, amino, mono- and di(C₁₋₆-alkyl)amino; carbamoyl, mono- and di(C₁₋₆-alkyl)aminocarbonyl, amino-C₁₋₆-alkyl-aminocarbonyl, mono- and di(C₁₋₆-alkyl)-amino-C₁₋₆-alkyl-aminocarbonyl, C₁₋₆-alkylcarbonylamino, cyano, guanidino, carbamido, C₁₋₆-alkanoyloxy, C₁₋₆-alkyl-sulphonyl-amino, aryl-sulphonyl-amino, heteroaryl-sulphonyl-amino, C₁₋₆-alkyl-suphonyl, C₁₋₆-alkyl-sulphinyl, C₁₋₆-alkylsulphonyloxy, nitro, sulphanyl, amino, amino-sulfonyl, mono- and di(C₁₋₆-alkyl)amino-sulfonyl, dihalogen-C₁₋₄-alkyl, trihalogen-C₁₋₄-alkyl and halogen, where aryl and heteroaryl representing substituents may be substituted 1-3 times with C₁₋₄-alkyl, C₁₋₄-alkoxy, nitro, cyano, amino or halogen, and any alkyl, alkoxy, and the like representing substituents may be substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino, or guanidine.

Preferably, the above-mentioned substituents are selected from hydroxy, C₁₋₆-alkyl, C₁₋₆-alkoxy, oxo (which may be represented in the tautomeric enol form), carboxy, C₁₋₆-alkylcarbonyl, formyl, amino, mono- and di(C₁₋₆-alkyl)amino; carbamoyl, mono- and di(C₁₋₆-alkyl)aminocarbonyl, amino-C₁₋₆-alkyl-aminocarbonyl, C₁₋₆-alkylcarbonylamino, guanidino, carbamido, C₁₋₆-alkyl-sulphonyl-amino, aryl-sulphonyl-amino, heteroaryl-sulphonyl-amino, C₁₋₆-alkyl-suphonyl, C₁₋₆-alkyl-sulphinyl, C₁₋₆-alkylsulphonyloxy, sulphanyl, amino, amino-sulfonyl, mono- and di(C₁₋₆-alkyl)amino-sulfonyl or halogen, where any alkyl, alkoxy and the like representing substituents may be substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine.

Especially preferred examples of such substituents are C₁₋₆-alkyl, C₁₋₆-alkoxy, amino, mono- and di(C₁₋₆-alkyl)amino, sulphanyl, carboxy or halogen, where any alkyl, alkoxy and the like representing substituents may be substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulponyl-amino or guanidine.

The term "salt thereof" is intended to mean a pharmaceutically acceptable acid addition salt obtainable by treating the base form of a functional group, such as an amine, with appropriate acids such as inorganic acids, for example hydrohalic acids; typically hydrochloric, hydrobromic, hydrofluoric or hydroiodic acid; sulfuric acid; nitric acid; phosphoric acid and the like; or organic acids, for example acetic, propionic, hydroacetic, 2-hydroxypropanoic acid, 2-oxopropanoic acid, ethandioic, propanedioic, butanedioic, (Z)-2-butenedioic, (E)-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic acid, cyclohexanesulfamic, 2-hydoxybenzoic, 4-amino-2-hydroxybenzoic, and other acids known to the skilled practitioner.

The term "pharmaceutically acceptable" when used in connection with the term "salt thereof" means that the salt does not cause any untoward effects in the patients to whom it is administered. Likewise, the term "pharmaceutically acceptable" when used in connection with the terms "carrier" and/or "excipient" means that the carrier and/or the excipient, at the dosages and with the concentrations employed, does not cause any untoward effects in the patients to whom it is administered.

In the present description and claims, any reference to "a" component, e.g. in the context of a substituent, etc., is intended to refer to one or more of such components, unless stated otherwise or unless it is clear from the particular context that this is not the case. For example, the expression "a component selected from the group consisting of A, B and C" is intended to include all combinations of A, B and C, i.e. A; B; C; A+B; A+C; B+C or A+B+C. The term "therapeutically effective amount" means a dosage or amount sufficient to produce a desired result. The desired result may comprise an objective or subjective improvement in the recipient of the dosage or amount.

A "prophylactic treatment" is a treatment administered to a subject who does not display signs or symptoms of a disease, pathology, or medical disorder, or displays only early signs or symptoms of a disease, pathology, or disorder, such that treatment is administered for the purpose of diminishing, preventing, or decreasing the risk of developing the disease, pathology, or medical disorder. A prophylactic treatment functions as a preventative treatment against a disease or disorder. A "prophylactic activity" is an activity of an agent, such as a compound disclosed herein, or a composition thereof, that, when administered to a subject who does not display signs or symptoms of pathology, disease or disorder, or who displays only early signs or symptoms of pathology, disease, or disorder, diminishes, prevents, or decreases the risk of the subject developing a pathology, disease, or disorder.

In the present context the term "therapeutic treatment", or simply "treatment", means a treatment administered to a subject who displays symptoms or signs of pathology, disease, or disorder, in which treatment is administered to the subject for the purpose of diminishing or eliminating those signs or symptoms of pathology, disease, or disorder. A "therapeutic activity" is an activity of an agent, such as a compound disclosed herein, or composition thereof, that eliminates or diminishes signs or symptoms of pathology, disease or disorder, when administered to a subject suffering from such signs or symptoms.

The term "subject" as used herein includes, but is not limited to, an organism; a mammal, including, e.g., a human being, non-human primate (e.g., baboon, orangutan, monkey), mouse, pig, cow, goat, cat, rabbit, rat, guinea pig, hamster, horse, monkey, sheep, or other non-human mammal; a non-mammal, including, e.g., a non-mammalian vertebrate, such as a bird (e.g., a chicken or duck) or a fish, and a non-mammalian invertebrate. In a preferred embodiment of the invention the subject is a being.

In the present context the term "tautomeric forms thereof" or "tautomer" refers to one of two or more structural isomers that exist in equilibrium and are readily converted from one isomeric form to another. Different tautomeric forms have the same molecular formula and are interchangeable forms involving the displacement of hydrogen atoms and electrons. Thus, it will be understood that when a compound of the invention is illustrated by its chemical structure, all possible tautomeric forms of the specifically depicted molecule are also within the scope of the present invention.

### The compound of the invention

As indicated previously, the present invention relates to a compound of the general formula (I) shown above. As can be seen from formula (I), the aminoguanidine substituent may be attached to the pyrrole ring at its position 2 or 3, i.e. the compounds of the general formula (Ia) and (Ib) merely differ from each other by the site of attachment to the pyrrole ring.

Accordingly, in another aspect the present invention relates to a compound of the general formula (Ia) or (Ib) 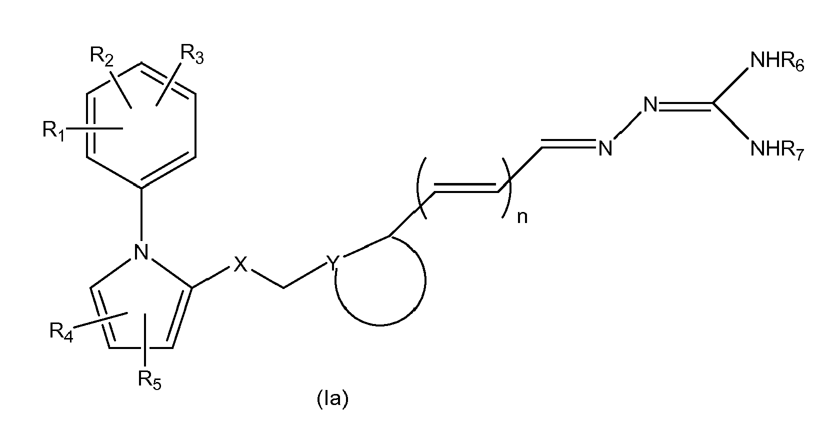 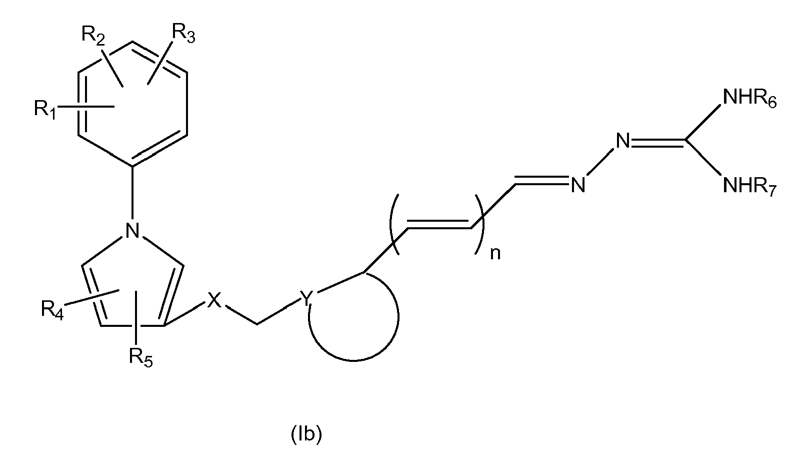 including tautomeric and isomeric forms, such as enantiomeric forms, diastereomeric forms and racemic forms, thereof,
wherein
X is (CH₂)ₘ, and m is 0, 1 or 2;
n is 0, 1 or 2;
Y is nitrogen (N) or carbon (C);
∪ represents, together with Y and the carbon atom covalently linked to Y, an optionally substituted five- or six-membered heterocyclyl or cycloalkyl group; each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen, where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine; each R₆ and R₇ is independently selected from the group consisting of hydrogen, optionally substituted C₁₋₆-alkyl, optionally substituted C₂₋₆-alkenyl, optionally substituted C₄₋₆-alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino-C₁₋₆-alkyl-aminocarbonyl and mono- and di(C₁₋₆-alkyl)amino-C₁₋₆-alkyl-aminocarbonyl; or R₆ and R₇ may together form a five- or six-membered nitrogen-containing ring; or a pharmaceutically acceptable salt thereof.

As discussed above, in the compounds of the general formula (Ia) the aminoguanidine substituent is attached to the pyrrole ring at position 2, whereas in the compounds of the general formula (Ib) the aminoguanidine substituent is attached to the pyrrole ring at position 3. In the following description, only compounds where the aminoguanidine substituent is attached to the pyrrole ring at position 2 is described with respect to preferred substituents, method for manufacturing, etc. It should be understood, however, that all statements made below with respect to the compounds of the invention where the aminoguanidine substituent is attached to the pyrrole ring at position 2 also apply to the compounds of the invention where the aminoguanidine substituent is attached to the pyrrole ring at position 3. Furthermore, the compounds of the general formula (I) herein are all shown in their trans isomeric forms. It should be understood, however, that the compounds of the general formula (I) may also be in their cis isomeric form. Thus, the configuration around a double bond in the molecule may be either cis or trans, although the trans configuration is preferred.

As will be understood by the skilled person the compounds of the general formula (I) may exist in the various tautomeric forms illustrated below (illustrated for compound (Ia) only).

Evidently, all possible tautomeric forms of the compounds of the invention are contemplated and hence included in the scope of the present invention. 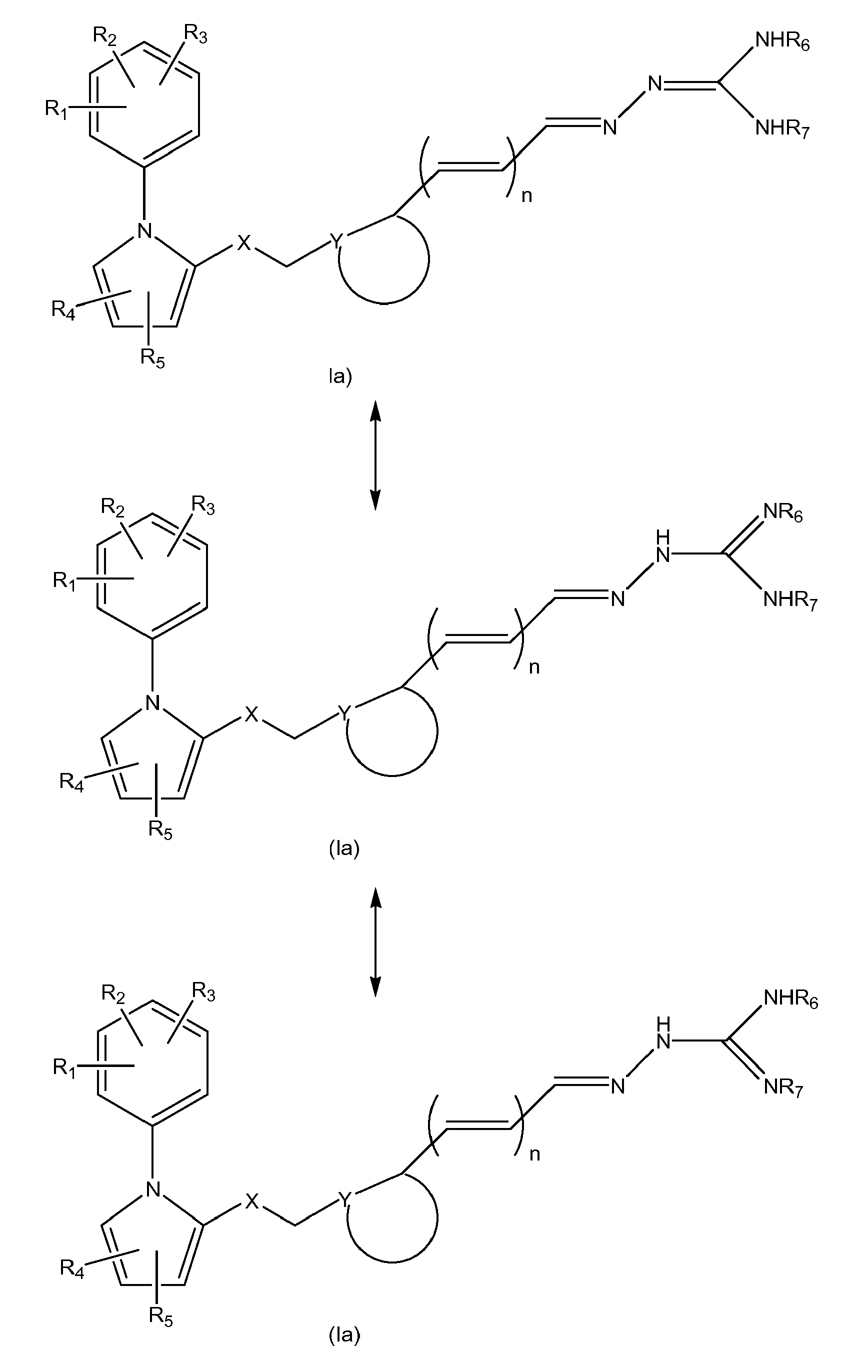 The compounds of the invention have basic properties and, consequently, they may be converted to their active acid addition salts by treatment with appropriate pharmaceutically acceptable acids. Examples of such acids include inorganic acids, such as hydrochloric acid, hydrobromic acid, hydrofluoric acid, hydroiodic acid, sulfuric acid, nitric acid, phosphoric acid and the like, or organic acids, such as acetic acid, propionic acid, hydroacetic acid, 2-hydroxypropanoic acid, 2-oxopropanoic acid, ethandioic acid, propanedioic acid, butanedioic acid, (Z)-2-butenedioic acid, (E)-butenedioic acid, 2-hydroxybutanedioic acid, 2,3-dihydroxybutanedioic acid, 2-hydroxy-1,2,3-propanetricarboxylic acid, methanesulfonic acid, ethanesulfonic acid, benzenesulfonic acid, 4-methylbenzenesulfonic acid, cyclohexanesulfamic acid, 2-hydoxybenzoic acid, 4-amino-2-hydroxybenzoic acid, and other acids known to the person skilled in the art.

The substituents R₁, R₂, R₃, R₄ and R₅ may be individually selected from the group of substituents indicated above. However, in a preferred embodiment of the invention each R₁, R₂, R₃, R₄ and R₅ is independently selected from the group consisting of hydrogen, optionally substituted C₁₋₆-alkyl, optionally substituted C₂₋₆-alkenyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen.

In a more preferred embodiment of the invention each R₁, R₂, R₃, R₄ and R₅ is independently selected from the group consisting of hydrogen, optionally substituted C₁₋₆-alkyl, optionally substituted C₂₋₆-alkenyl, hydroxy, optionally substituted C₁₋₆-alkoxy, amino, cyano, nitro and halogen, such as bromo, chloro and fluoro. Specific examples of highly preferred (non-substituted) C₁₋₆-alkyl groups include C₁₋₄-alkyl, such as methyl or ethyl, in particular methyl. Specific examples of highly preferred substituted C₁₋₆-alkyl groups include substituted C₁₋₄-alkyl, such as halogen-substituted C₁₋₄-alkyl, e.g. trihalo-C₁₋₄-alkyl, in particular tribromomethyl, trichloromethyl and trifluoromethyl among which trichloromethyl and trifluoromethyl are particularly preferred. Specific examples of highly preferred (non-substituted) C₂₋₆-alkenyl groups include C₂₋₄-alkenyl, such as vinyl, allyl and butenyl, in particular allyl. Specific examples of highly preferred (non-substituted) C₁₋₆-alkoxy groups include C₁₋₄-alkoxy, such as methoxy or ethoxy, in particular methoxy.

Concerning the substituents R₆ and R₇, these substituents may each be independently selected from the group consisting of hydrogen, optionally substituted C₁₋₆-alkyl, optionally substituted C₂₋₆-alkenyl, optionally substituted C₄₋₆-alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono-and di(C₁₋₆-alkyl)aminocarbonyl, amino-C₁₋₆-alkyl-aminocarbonyl and mono- and di(C₁₋₆-alkyl)amino-C₁₋₆-alkyl-aminocarbonyl; or R₆ and R₇ may together form a five- or six-membered nitrogen-containing ring. In a preferred embodiment of the invention, at least one of R₆ and R₇ is hydrogen. In a particular preferred embodiment of the invention R₆ and R₇ are both hydrogen, i.e. the compound of the invention has the structure shown in the general formula (II): 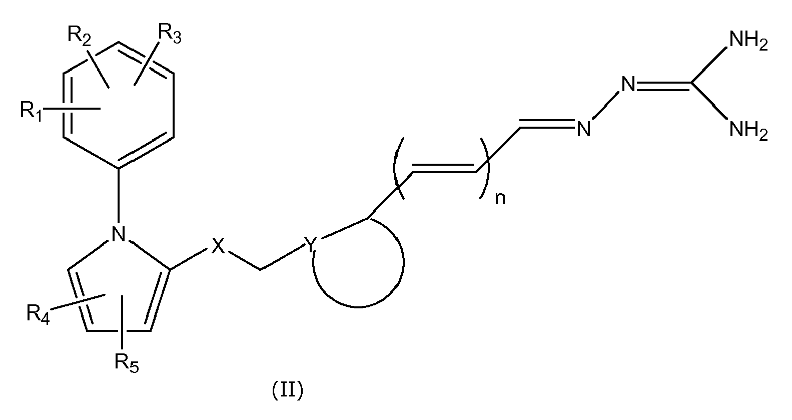 In an interesting embodiment of the invention, R₄ is hydrogen and R₁, R₂, R₃ and R₅ are as defined above. Thus, according to this embodiment of the invention, the compound of the invention has the structure shown in the general formula (III): 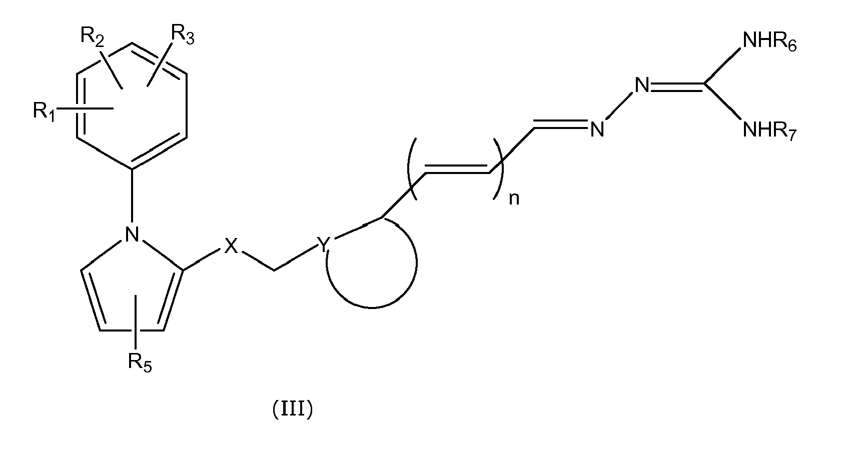 wherein each of the substituents are as defined above. In particular, it is preferred that both of R₆ and R₇ are hydrogen.

In a further interesting embodiment of the invention, R₁ and R₄ are hydrogen and R₂, R₃ and R₅ are as defined above. Thus, according to this embodiment of the invention, the compound of the invention has the structure shown in the general formula (IV): 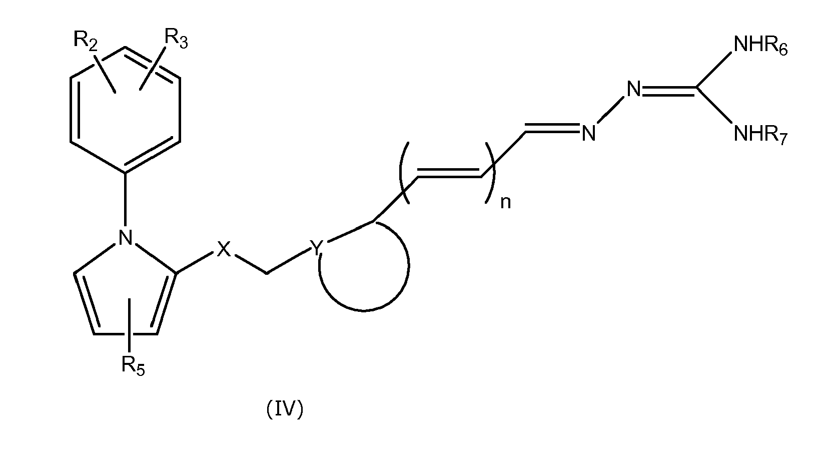 wherein each of the substituents are as defined above. In particular, it is preferred that both of R₆ and R₇ are hydrogen.

In a preferred embodiment of the invention, R₁, R₄ and R₅ are hydrogen and R₂ and R₃ are as defined above. Thus, according to this embodiment of the invention, the compound of the invention has the structure shown in the general formula (V): 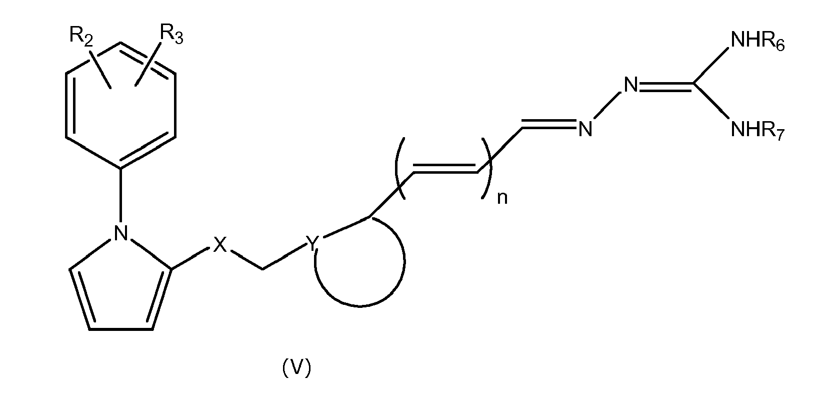 wherein each of the substituents are as defined above. In particular, it is preferred that both of R₆ and R₇ are hydrogen.

Concerning the compounds described above in connection with the general formulae (I), (II), (III), (IV) and (V) it will be understood that the individual substituents may be attached to the ring systems at different positions. More particularly, and with reference to the general formula (V) above, the attachment of the R₂ and R₃ may be as follows: In one embodiment of the invention R₂ is located in the 2-position and R₃ is located in the 3-position. In another embodiment of the invention R₂ is located in the 2-position and R₃ is located in the 4-position. In yet another embodiment of the invention R₂ is located in the 2-position and R₃ is located in the 5-position. In a further embodiment of the invention R₂ is located in the 2-position and R₃ is located in the 6-position. In a still further embodiment of the invention R₂ is located in the 3-position and R₃ is located in the 4-position. In an even further embodiment of the invention R₂ is located in the 3-position and R₃ is located in the 5-position. In yet another embodiment of the invention R₂ is located in the 3-position and R₃ is located in the 6-position.

In another preferred embodiment of the invention, R₁, R₂, R₄ and R₅ are hydrogen and R₃ is as defined above. Thus, according to this embodiment of the invention, the compound of the invention has the structure shown in the general formula (VI): 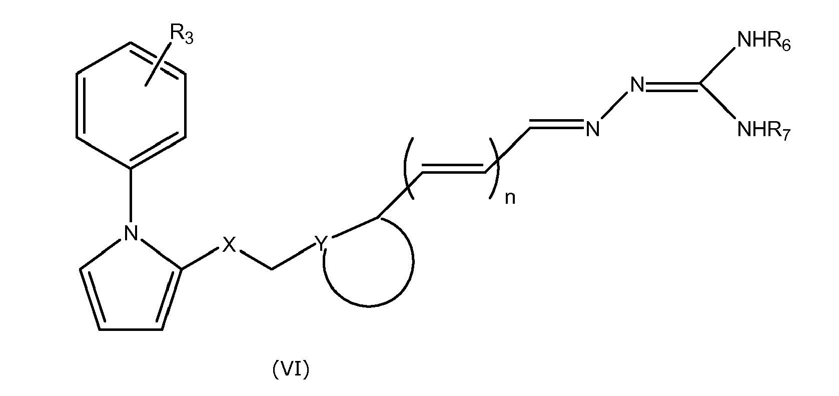 wherein each of the substituents are as defined above. In particular, it is preferred that both of R₆ and R₇ are hydrogen.

In one embodiment of the invention R₃ is located in the 2-position. In another embodiment of the invention R₃ is located in the 3-position. In yet another embodiment of the invention R₃ is located in the 4-position.

In a still further interesting embodiment of the invention all of R₁, R₂, R₃, R₄ and R₅ are hydrogen.

As indicated above, X is (CH₂)ₘ, and m is 0, 1 or 2. In a preferred embodiment, m is 0, i.e. according to this embodiment of the invention, the compound of the invention has the structure shown in the general formula (VII): 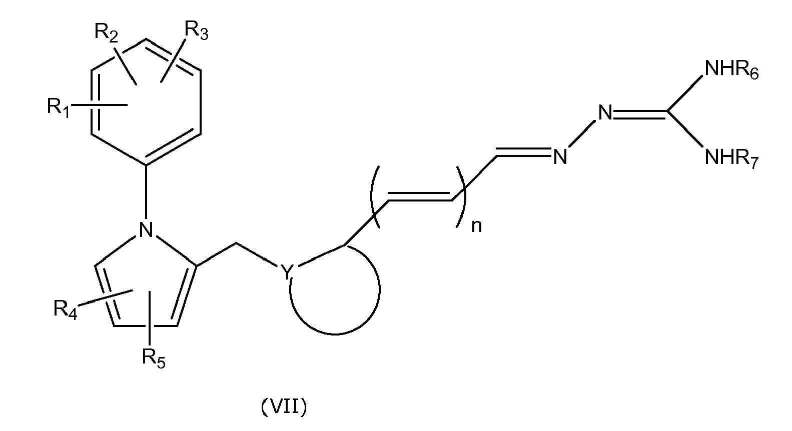 wherein each of the substituents are as defined above. In particular, it is preferred that both of R₆ and R₇ are hydrogen. In another preferred embodiment, Y is nitrogen (N). In yet another preferred embodiment, n is 0. In still a further preferred embodiment, R₆ and R₇ are hydrogen, Y is nitrogen (N), and n is 0.

As also indicated above, n is an integer of 0, 1 or 2. In a preferred embodiment of the invention n is 0 or 1. In the most preferred embodiment of the invention n is 0. Thus, according to this embodiment of the invention, the compound of the invention has the structure shown in the general formula (VIII): 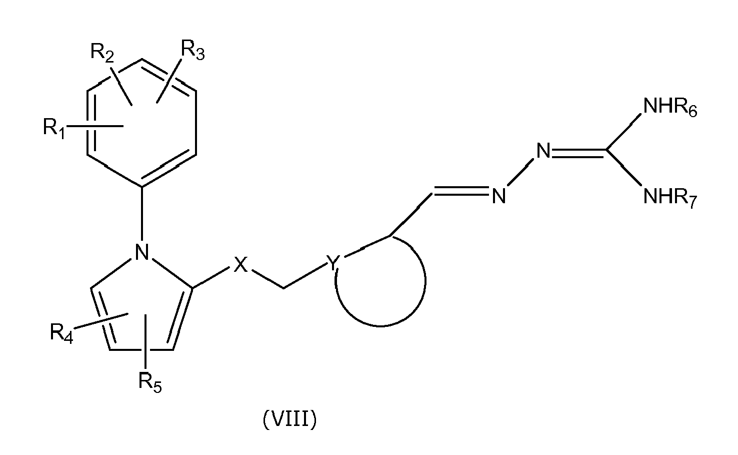 Concerning Y, which forms part of the ring system present in the aminoguanidine substituent, this atom may be a carbon atom or a nitrogen atom, i.e. ∪ represents, together with Y and the carbon atom covalently linked to Y, an optionally substituted five-or six-membered heterocyclyl or cycloalkyl group. As will be understood, the molecule contains, in case Y is nitrogen, a single asymmetric carbon atom. Thus, in this embodiment of the invention the molecule of invention may be in either its enantiomeric S-form, its enantiomeric R-form, or the molecule of the invention may be a racemic mixture. Likewise, the molecule contains, in case Y is carbon, two asymmetric carbon atoms. Thus, in this embodiment of the invention the molecule of invention may be in either its SS-form, its RR-form, its SR-form, its RS-form, or any mixture thereof.

In an interesting embodiment of the invention Y is carbon (C), i.e. ∪ represents, together with Y and the carbon atom covalently linked to Y, an optionally substituted five- or six-membered cycloalkyl group. Accordingly, in one embodiment of the invention ∪ represents, together with Y and the carbon atom covalently linked to Y, an optionally substituted six-membered cycloalkyl group as illustrated below: 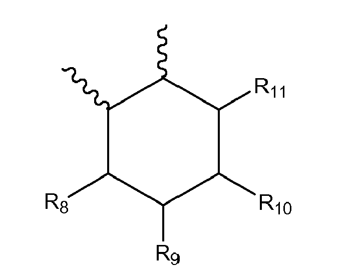 wherein each of R₈, R₉, R₁₀, and R₁₁ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen,
where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine.

Preferably, each of R₈, R₉, R₁₀, and R₁₁ is independently selected from the group consisting of hydrogen, optionally substituted C₁₋₆-alkyl, optionally substituted C₂₋₆-alkenyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen.

More preferably, each of R₈, R₉, R₁₀, and R₁₁ is independently selected from the group consisting of hydrogen, optionally substituted C₁₋₆-alkyl, optionally substituted C₂₋₆-alkenyl, hydroxy, optionally substituted C₁₋₆-alkoxy, amino, cyano, nitro and halogen. In a particular interesting embodiment of the invention all of R₈, R₉, R₁₀, and R₁₁ are hydrogen.

In another embodiment of the invention ∪ represents, together with Y and the carbon atom covalently linked to Y, an optionally substituted five-membered cycloalkyl group as illustrated below: 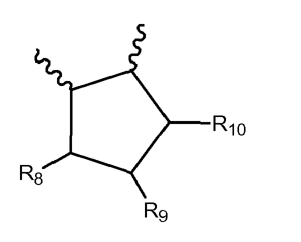 wherein each of R₈, R₉ and R₁₀ is as defined above. Again, in a particular interesting embodiment of the invention all of R₈, R₉ and R₁₀ are hydrogen.

In a preferred embodiment of the invention Y is nitrogen (N), i.e. ∪ represents, together with Y and the carbon atom covalently linked to Y, an optionally substituted five- or six-membered heterocyclyl group. Any of the heterocyclyl groups mentioned in connection with the definition of this term may be used for this purpose. However, in one embodiment of the invention u represents, together with Y and the carbon atom covalently linked to Y, an optionally substituted six-membered heterocyclyl group as illustrated below: 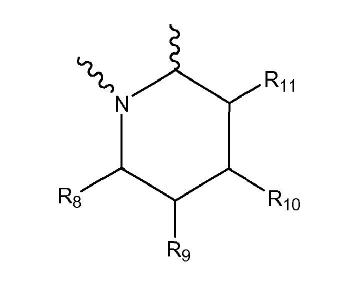 wherein each of R₈, R₉, R₁₀, and R₁₁ is as defined above. Also in this embodiment of the invention is it preferred that all of R₈, R₉, R₁₀ and R₁₁ are hydrogen.

In an even more preferred embodiment of the invention, ∪ represents, together with Y and the carbon atom covalently linked to Y, an optionally substituted five-membered heterocyclyl group as illustrated below: 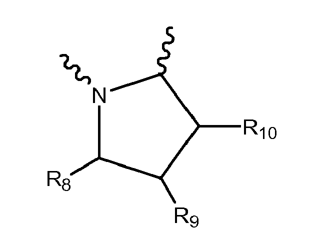 wherein each of R₈, R₉ and R₁₀ are as defined above. In the most preferred embodiment of the invention, all of R₈, R₉ and R₁₀ are hydrogen.

It should be understood that all of the above statements made in connection with the compounds of the invention apply equally well to compounds of the invention where the aminoguanidine substituent is attached to the pyrrole ring at position 2 or 3 (although usually only illustrated and discussed for compounds of the invention where the aminoguanidine substituent is attached to the pyrrole ring at position 2). Nevertheless, in a preferred embodiment of the invention the aminoguanidine substituent is attached to the pyrrole ring at position 2, i.e. in a preferred embodiment the compounds of the invention has the stereochemistry indicated in the general formula (Ia) and the formulae (II)-(VIII) above.

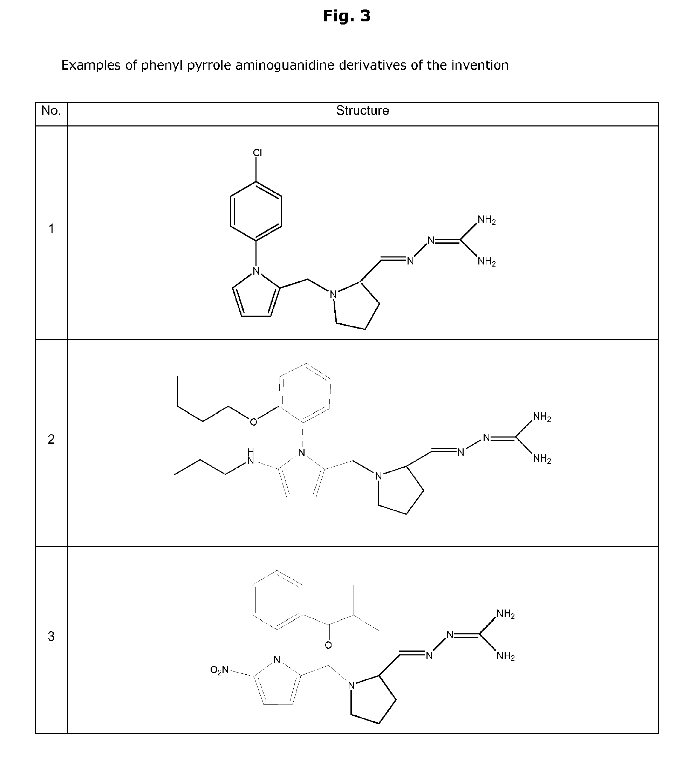

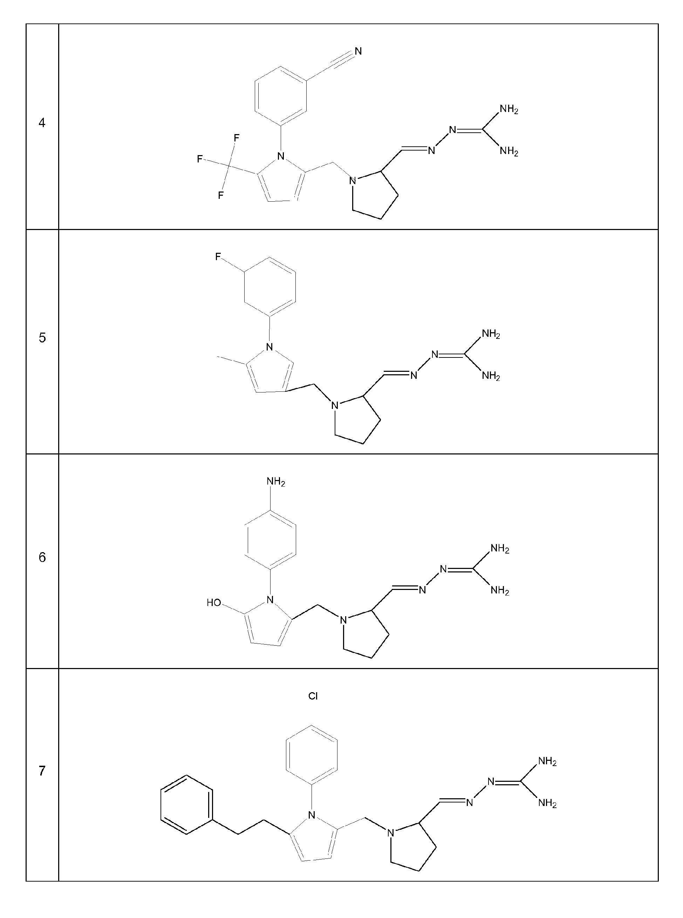

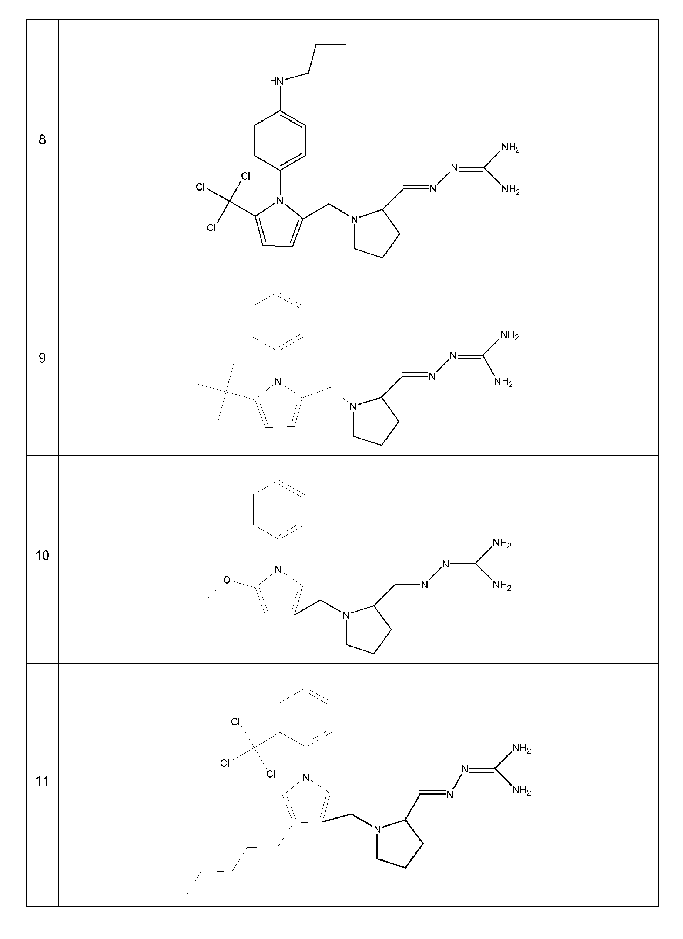

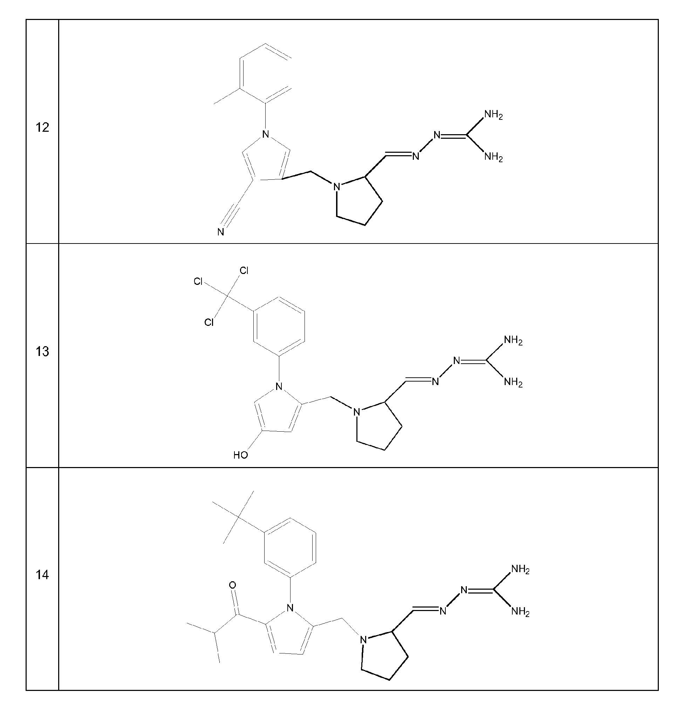

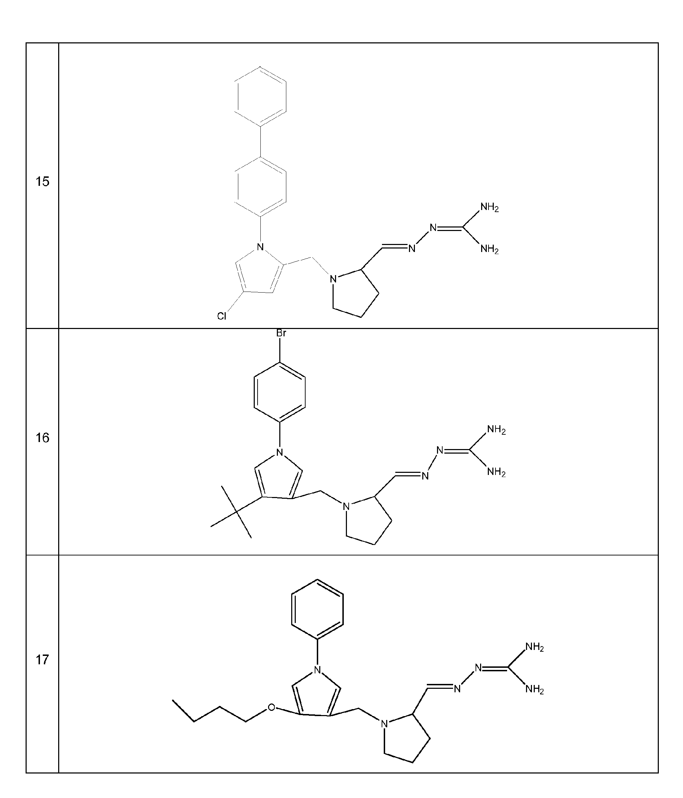

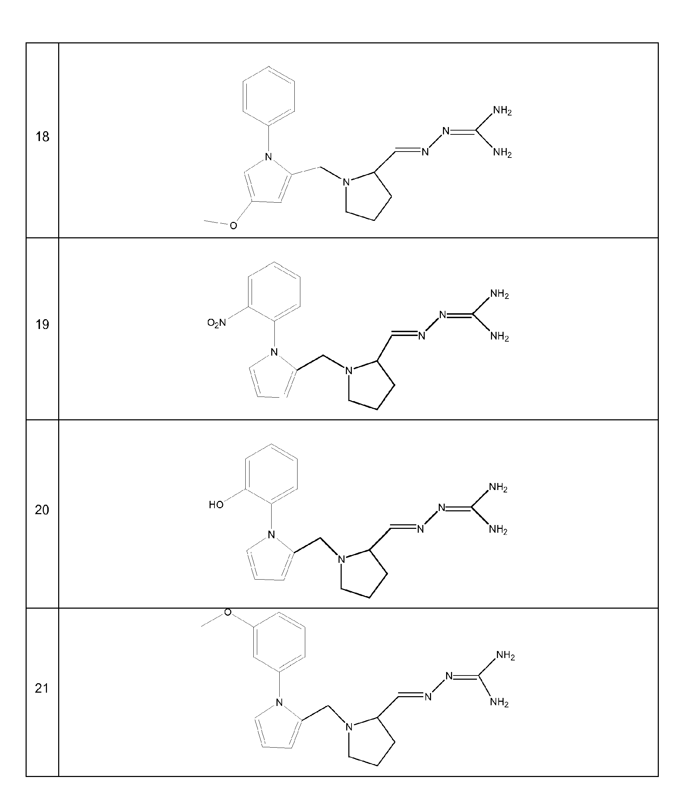

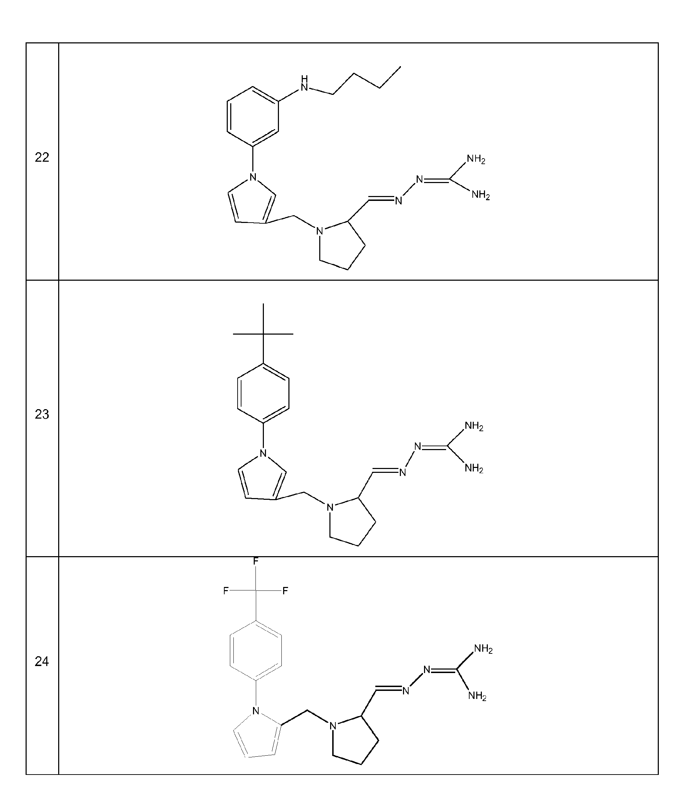

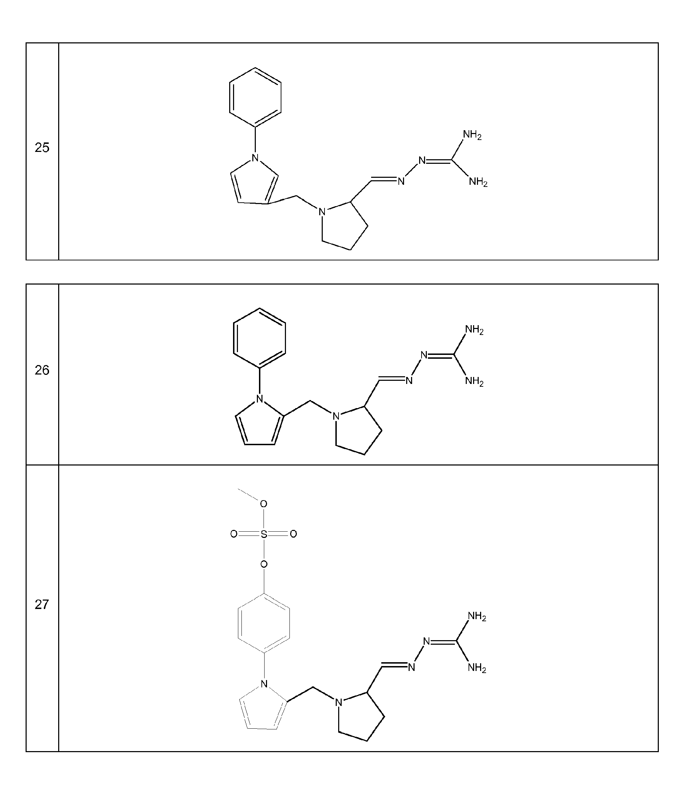

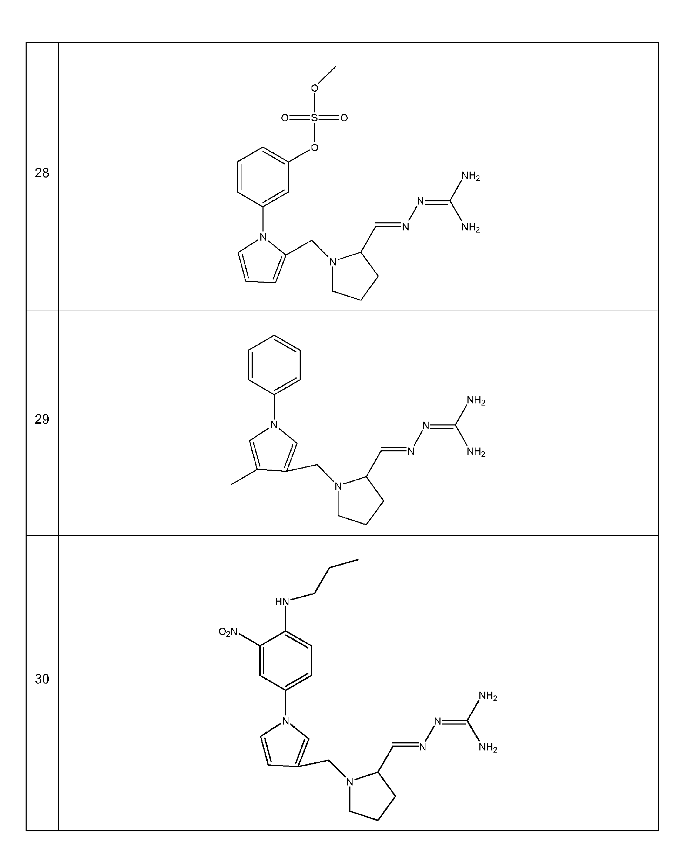

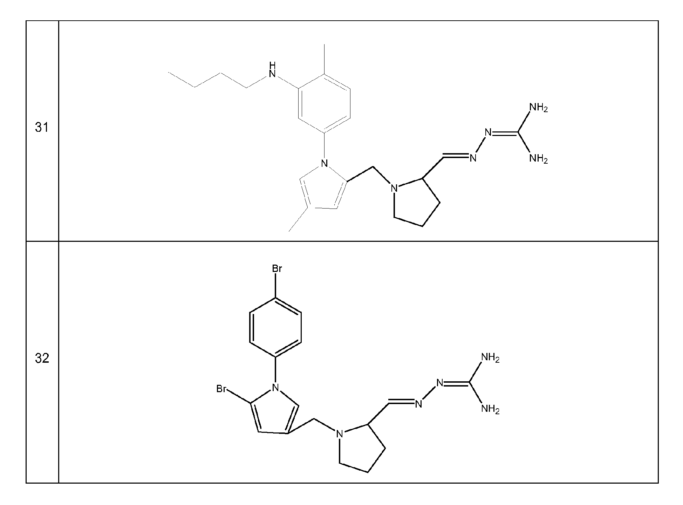

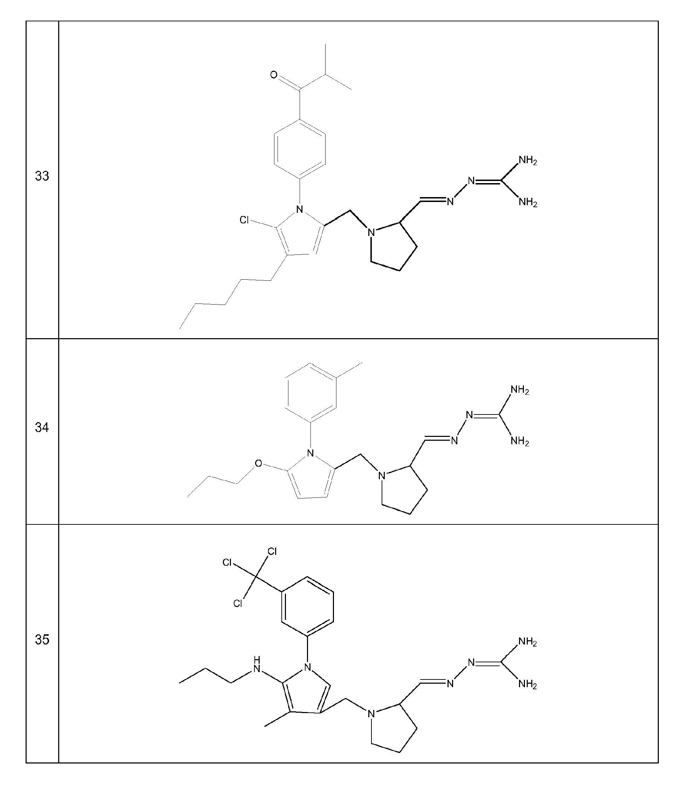

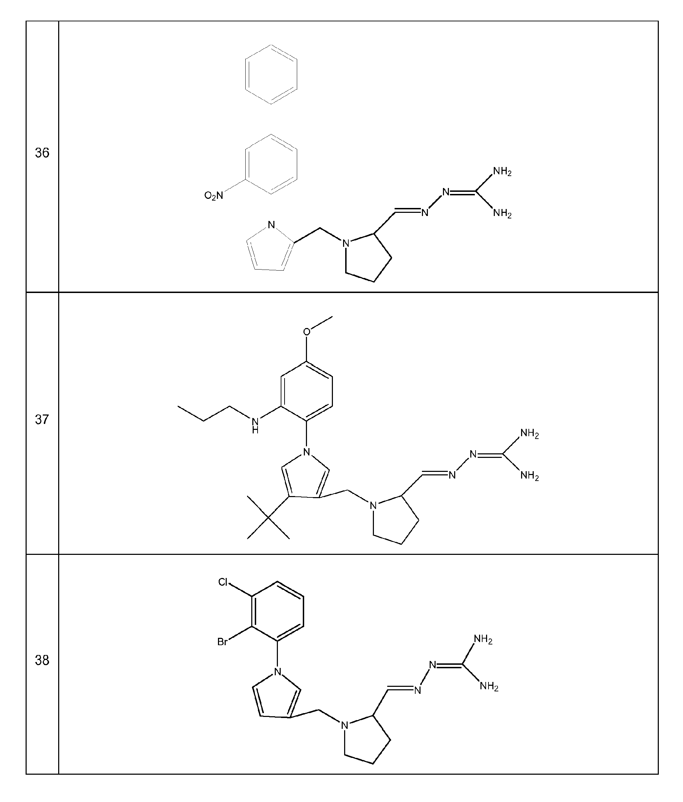

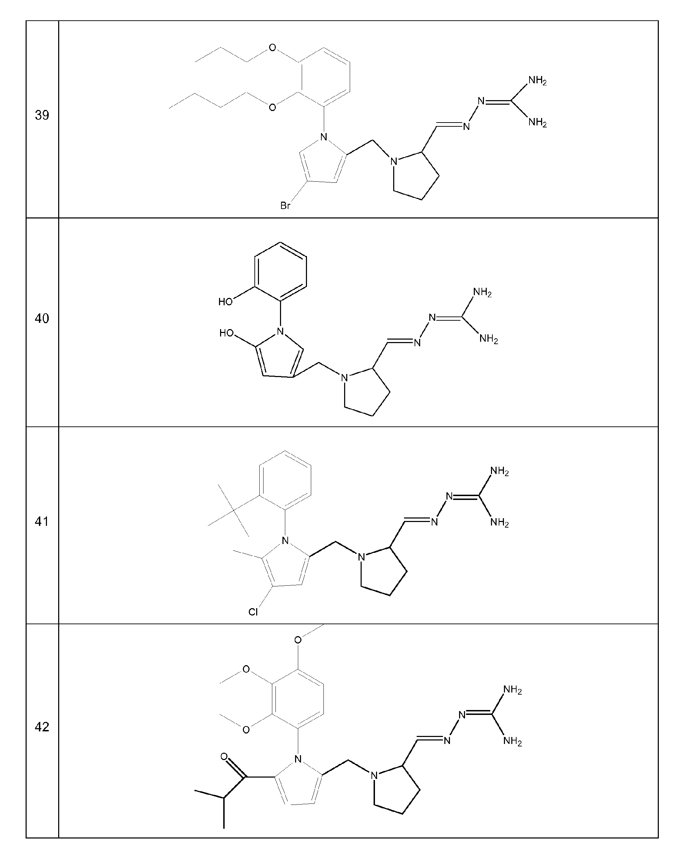

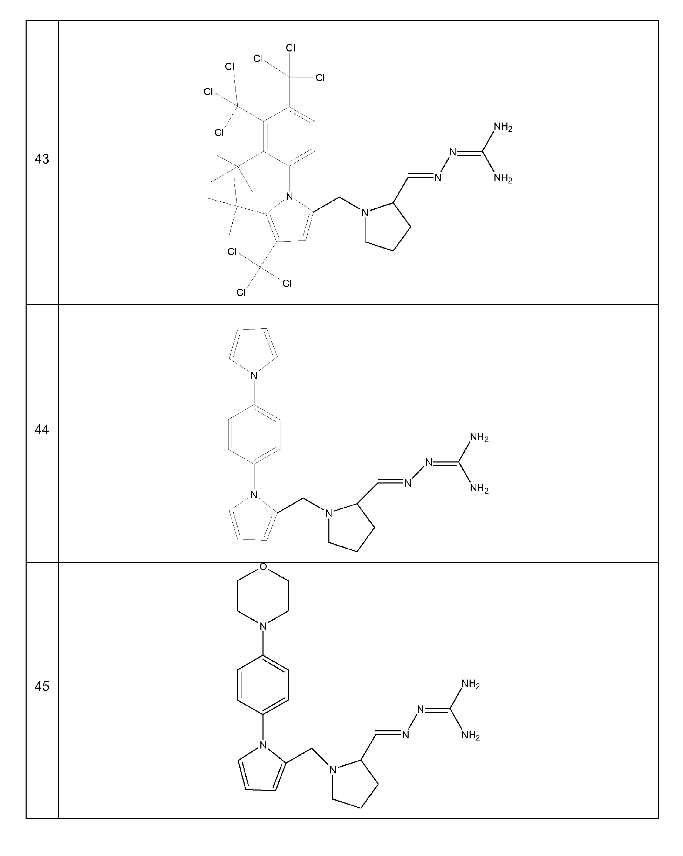

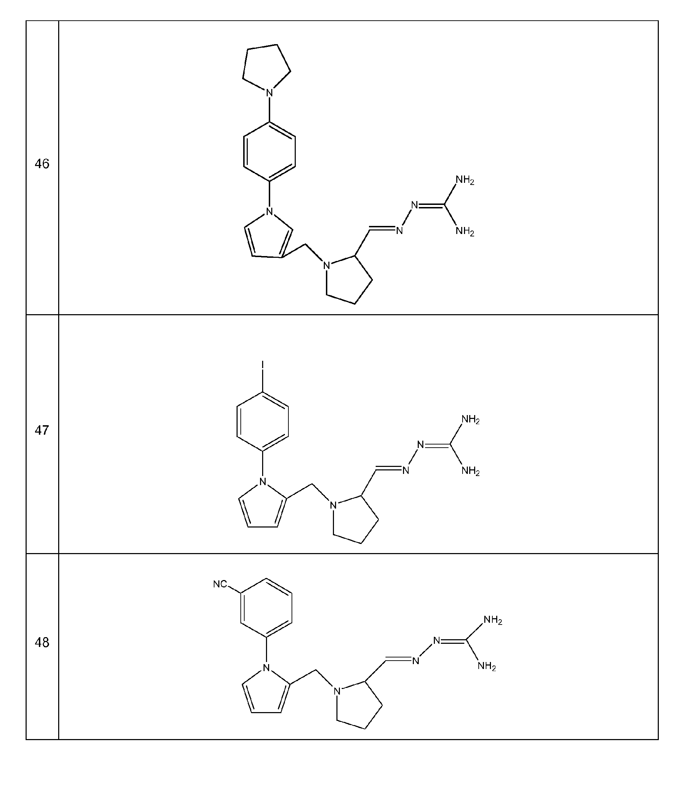

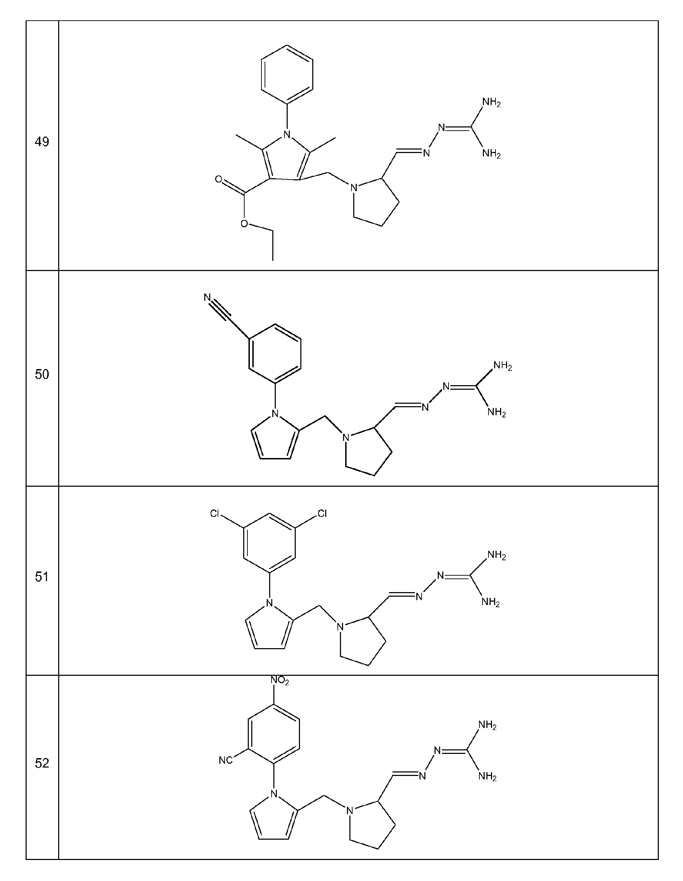

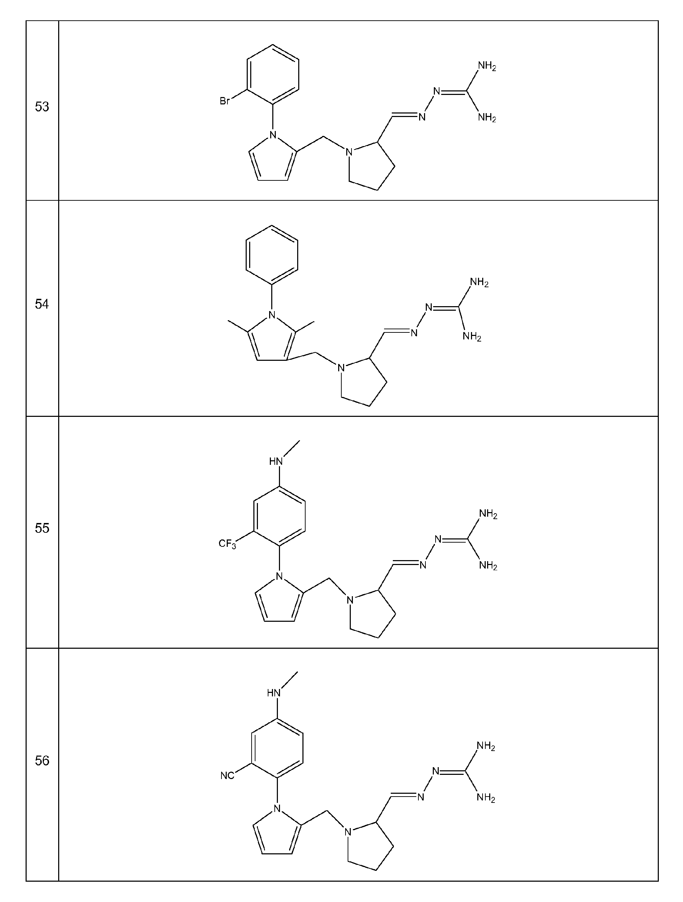

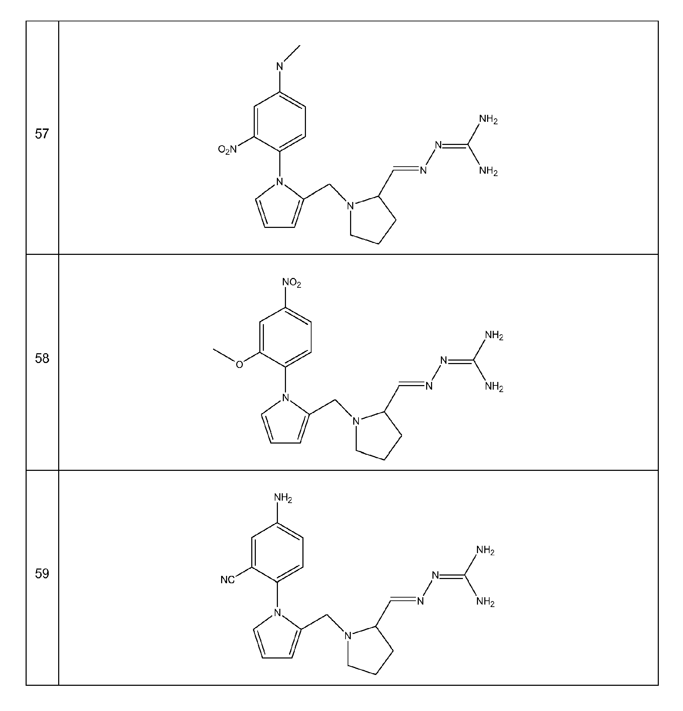

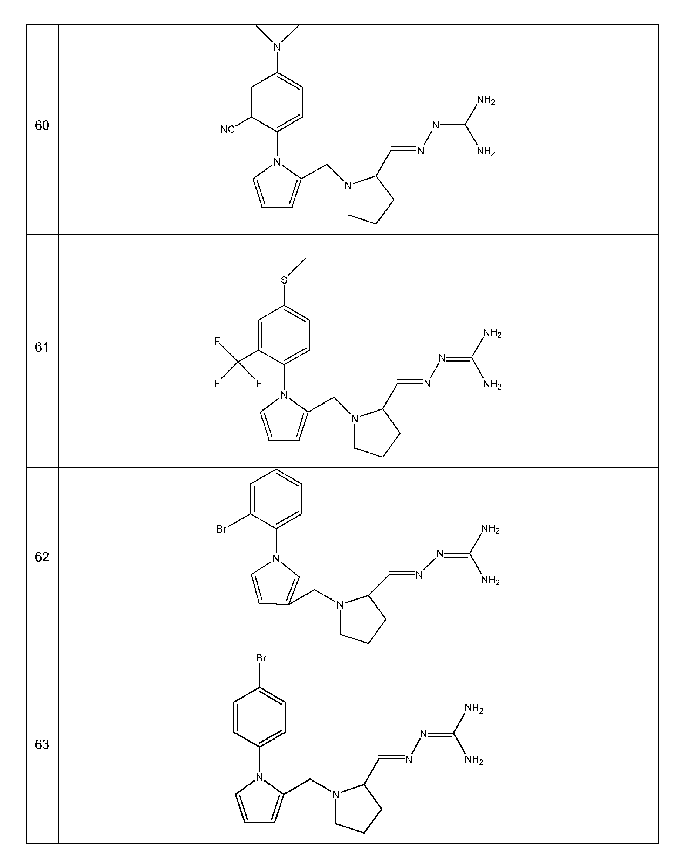

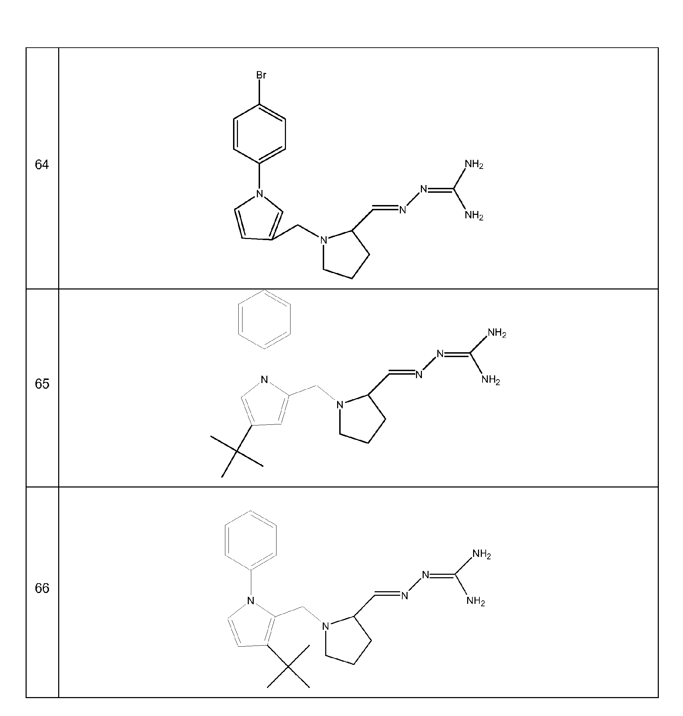

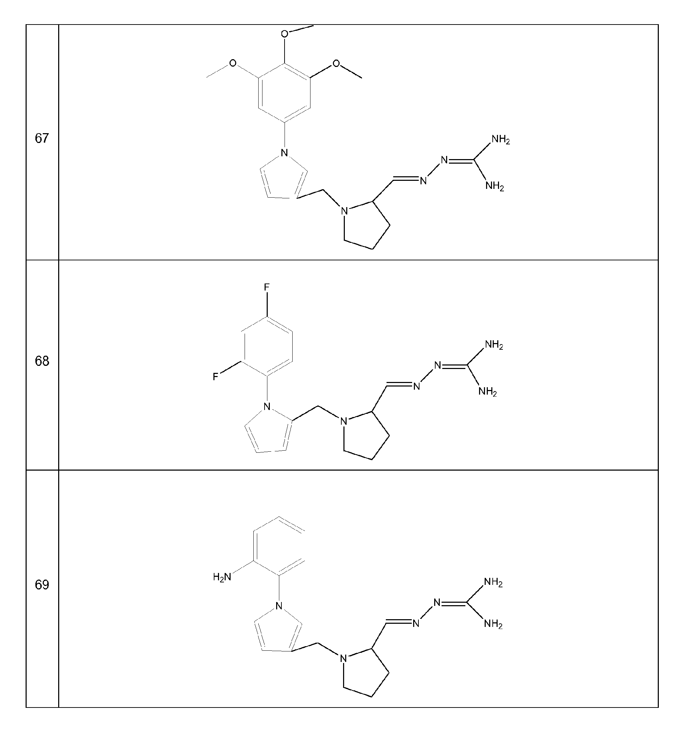

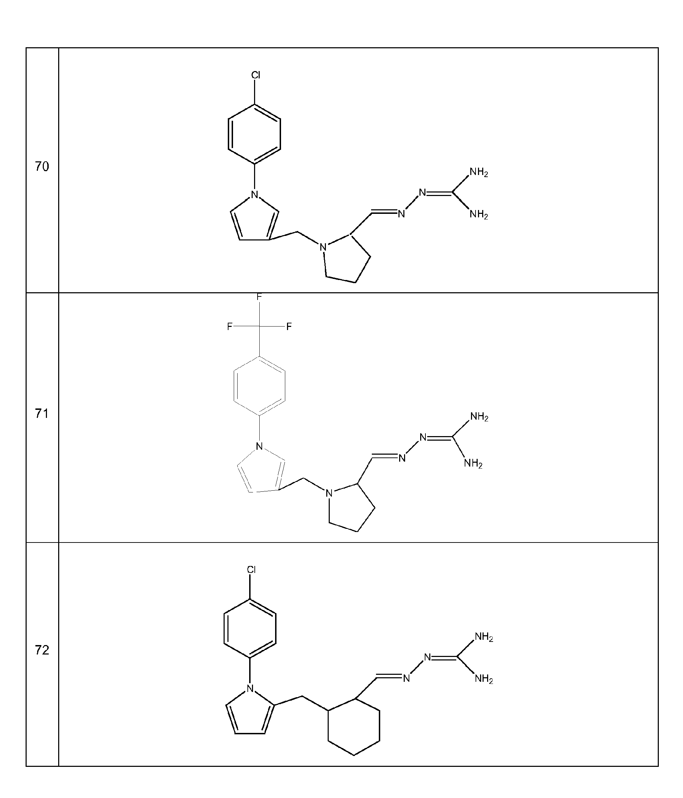

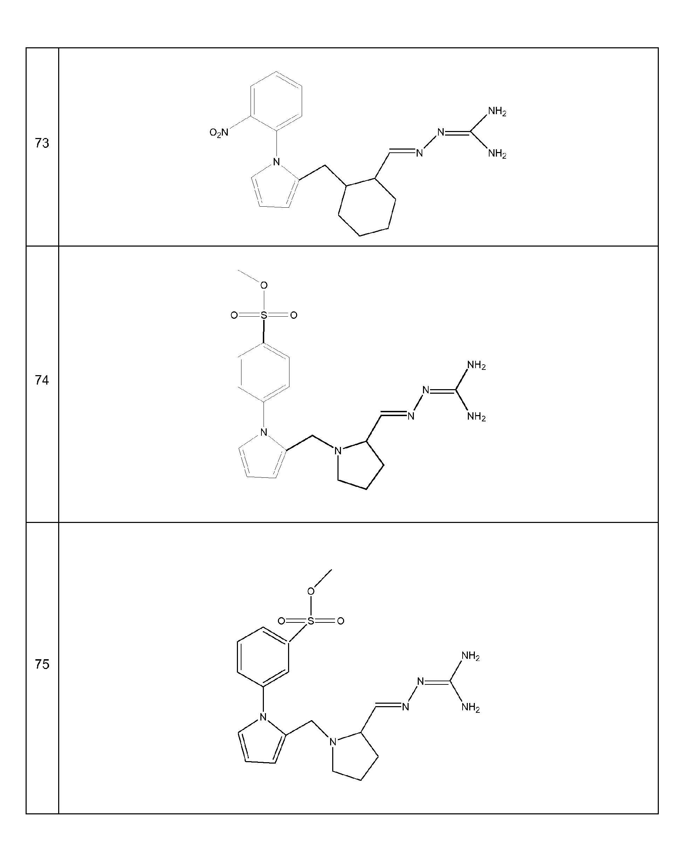

Compounds according to the invention which are currently believed to be of particular interest are shown in Fig. 3.

### Methods of preparing the compounds of the invention

The compounds of the invention may be prepared by standard methods known to the person skilled in the art. Thus, a compound of the general formula (Ia) or (Ib) above may be prepared essentially as described in WO 03/013509.

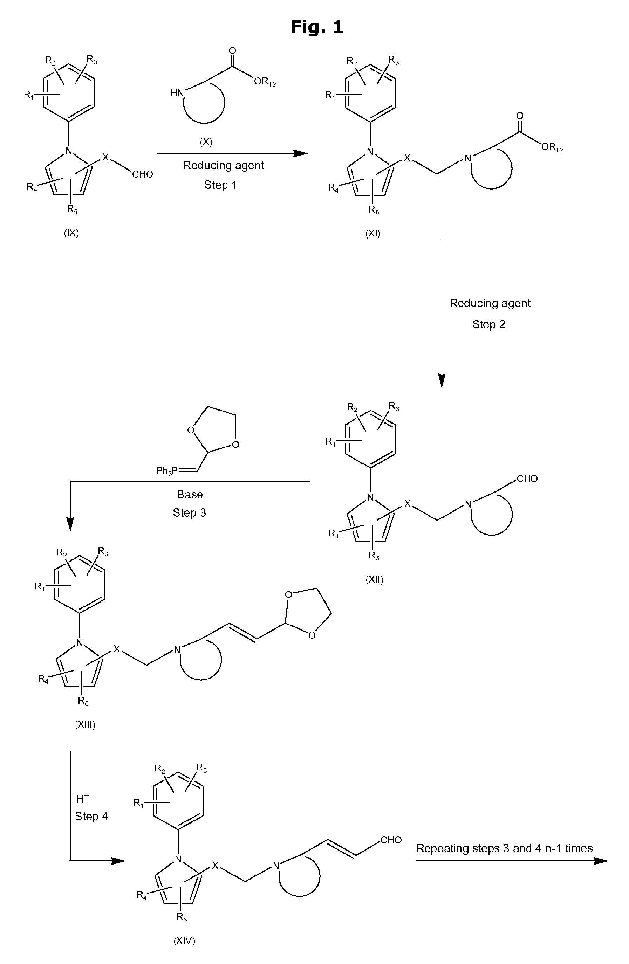

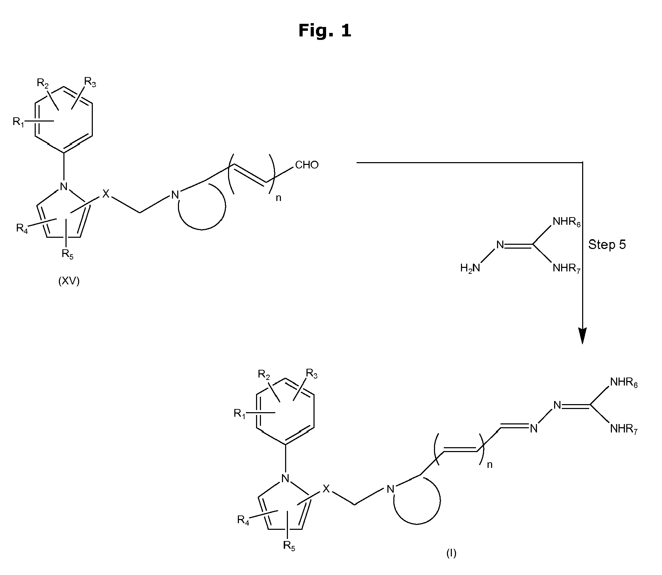

More particularly, in case Y=N the compound of the invention may be prepared as illustrated in Fig. 1.

Referring to Fig. 1, an aldehyde of the general formula (IX) is reacted with the amino acid ester (X), where X may be in its S-form, R-form or a racemic mixture, in a suitable solvent (Step 1). The amino acid ester (X) is typically an amino acid C₁₋₆-alkyl ester, i.e. R₁₂ is C₁₋₆-alkyl, preferably methyl or ethyl. Step 1 is carried out in the presence of a suitable reducing agent, such as sodium cyanoborohydride, sodium triacetoxy borohydride, sodium borohydride, or hydrogen in the presence of a suitable catalyst such as palladium or carbon, in a suitable organic solvent to yield an ester of the general formula (XI).

In Step 2, the obtained ester (XI) may be reduced to its corresponding aldehyde (XII) using a suitable reducing agent, such as lithium aluminium hydride in a suitable organic solvent, such as diethyl ether or THF.

The aldehyde (XII) is then converted to the chain-elongated aldehyde (XIV), via the intermediate (XIII), by the well-known Wittig reaction (Steps 3 and 4).

Formation of the intermediate compound (XIII) is carried out in a suitable organic solvent, typically a protic solvent, such as dimethylsulfoxide, dimethylformamide, hexamethyl-phosphorotriamide, in the presence of a strong base, such as an alkoxide, e.g. sodium or potassium tert-butoxide (Step 3). The intermediate (XIII) is subsequently converted to the desired aldehyde (XIV) by acidic hydrolysis using standard methods (Step 4). As will be understood, Steps 3 and 4 may be repeated until the desired chain length (n) has been obtained. Thus, after repeated cycles of Steps 3 and 4, the aldehyde (XV) is obtained.

The aldehyde (XV) is then converted to the desired end-product (I) by reacting the aldehyde (XV) with an aminoguanidine derivative of the below general formula in a suitable organic solvent: 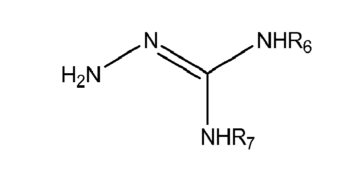 wherein the individual substitutents have the same meaning as described above. Preferably, the aldehyde (XV) is reacted with an aminoguanidine derivative of the above general formula where the aminoguanidine derivative is in the form of an acid addition salt, such as the bicarbonate salt.

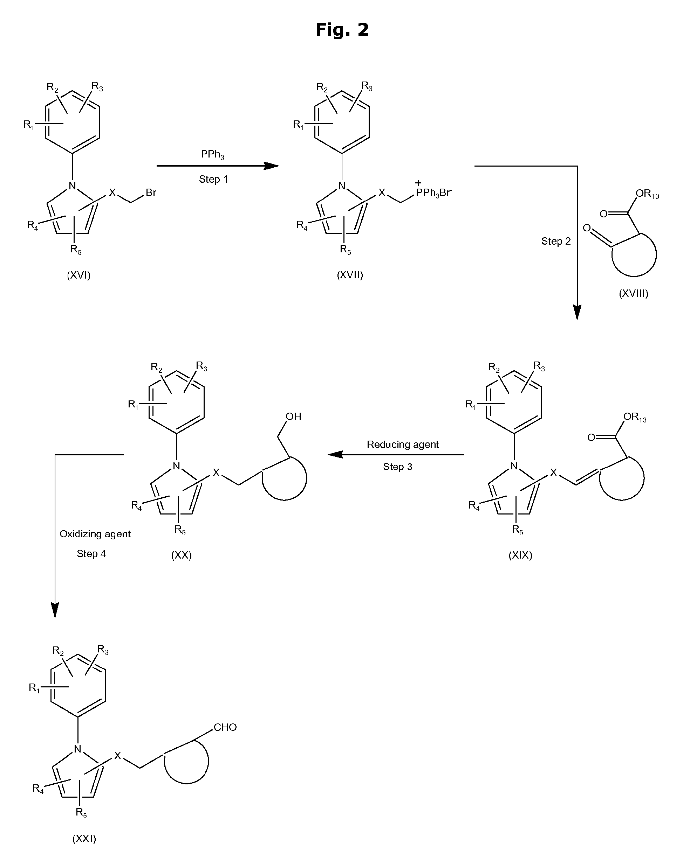

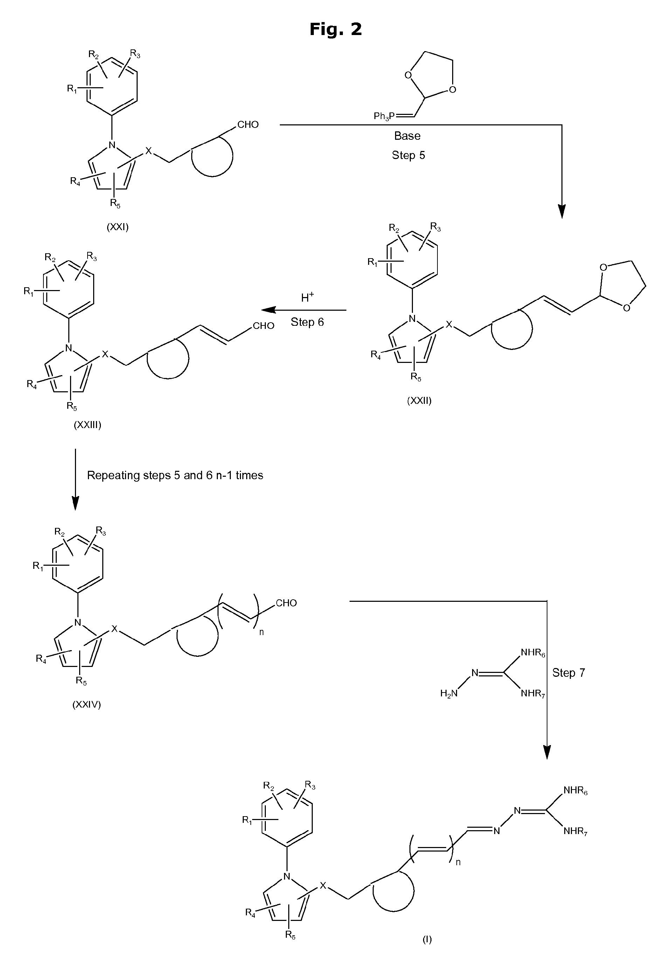

In case Y=C the compound of the invention may be prepared as illustrated in Fig. 2.

Referring to Fig. 2, a halide, such as the bromide, of the general formula (XVI) is transformed into a Wittig reagent of the general formula (XVII) by standard methods by reaction with triphenyl phoshpine in a suitable solvent (Step 1). In the well-known Wittig reaction, the Wittig reagent (XVII) is reacted with a cyclic ketoester of the general formula (XVIII), cf. Step 2 of Fig. 2, to yield the ester (XIX). The cyclic ketoester (XVIII) is typically a C₁₋₆-alkyl ester, i.e. R₁₃ is C₁₋₆-alkyl, preferably methyl or ethyl.

The thus obtained ester (XIX) is transformed to its corresponding aldehyde (XXI), via the alcohol intermediate (XX), using standard methods known to the person skilled in the art (Steps 3 and 4). Step 3 is conveniently carried out using a suitable reducing agent, such as lithium aluminium hydride in a suitable organic solvent, such as diethyl ether or THF. The alcohol intermediate (XX) is subsequently oxidised to its corresponding aldehyde (XXI) by means of a suitable oxidising agent, such as sulfur trioxide pyridine complex or oxalylchloride, dimethyl sulfoxide and triethyl amine (Swern oxidation) (Step 4).

The obtained aldehyde (XXI) corresponds to the aldehyde (XII) described above in the case where Y is nitrogen. Accordingly, Steps 5, 6 and 7 shown in Fig. 2 (where Y=C) may be conducted in a similar way as Steps 3, 4 and 5 (where Y=N) described above.

### Pharmaceutical compositions

The compound of the invention is preferably administered in a composition including a pharmaceutically acceptable carrier or excipient. The term "pharmaceutically acceptable" means a carrier or excipient that does not cause any untoward effects in patients to whom it is administered. Such pharmaceutically acceptable carriers and excipients are well known in the art (Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]).
The exact dose to be administered depends on the circumstances. Normally, the dose should be capable of preventing or lessening the severity or spread of the condition or indication being treated. It will be apparent to those of skill in the art that an effective amount of the compound of the invention depends, *inter alia,* upon the disease, the dose, the administration schedule, whether the compound of the invention is administered alone or in conjunction with other therapeutic agents, the general health of the patient, and the like. Generally, and in particular if administered via the oral route, the compound of the invention should be administered in a dose of 0.1 to 100 mg body weight per kilo throughout the treatment period.

The pharmaceutical composition may be formulated in a variety of forms, including liquid, gel, lyophilised, powder, compressed solid, or any other suitable form. The preferred form will depend upon the particular indication being treated and will be apparent to one of skill in the art.

The pharmaceutical composition may be administered orally, subcutaneously, intravenously, intracerebrally, intranasally, transdermally, intraperitoneally, intramuscularly, intrapulmonary, vaginally, rectally, intraocularly, or in any other acceptable manner, e.g. using PowderJect or ProLease technology. The composition can be administered continuously by infusion, although bolus injection is acceptable, using techniques well known in the art, such as pumps or implantation. In some instances the composition may be directly applied as a solution or spray. The preferred mode of administration will depend upon the particular indication being treated and will be apparent to one of skill in the art. However, the currently preferred mode of administration is via the oral route.

The pharmaceutical composition of the invention may be administered in conjunction with other therapeutic agents. These agents may be incorporated as part of the same pharmaceutical composition or may be administered separately from the composition of the invention, either concurrently or in accordance with any other acceptable treatment schedule.

### Oral administration

For oral administration, the pharmaceutical composition may be in solid or liquid form, e.g. in the form of a capsule, tablet, suspension, emulsion or solution. The pharmaceutical composition is preferably made in the form of a dosage unit containing a given amount of the active ingredient. A suitable daily dose for a human or other mammal may vary widely depending on the condition of the patient and other factors, but can be determined by persons skilled in the art using routine methods.

Solid dosage forms for oral administration may include capsules, tablets, suppositories, powders and granules. In such solid dosage forms, the active compound may be admixed with at least one inert diluent such as sucrose, lactose, or starch. Such dosage forms may also comprise, as is normal practice, additional substances, e.g. lubricating agents such as magnesium stearate. In the case of capsules, tablets and pills, the dosage forms may also comprise buffering agents. Tablets and pills can additionally be prepared with enteric coatings.

The compound of the invention may be admixed with adjuvants such as lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinyl-pyrrolidine, and/or polyvinyl alcohol, and tableted or encapsulated for conventional administration. Alternatively, the compound of the invention may be dissolved in saline, water, polyethylene glycol, propylene glycol, ethanol, oils (such as corn oil, peanut oil, cottonseed oil or sesame oil), tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The pharmaceutical compositions may be subjected to conventional pharmaceutical operations such as sterilisation and/or may contain conventional adjuvants such as preservatives, stabilisers, wetting agents, emulsifiers, buffers, fillers, etc.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, sweeteners, flavoring agents and perfuming agents.
The invention also relates to processes for the manufacture of and pharmaceutical preparations comprising one or more of the compounds of the invention, as well as to their uses for various medical and veterinary practices related to melanocyte stimulating hormone receptors.

### Therapeutic use

The compounds of the present invention have surprisingly been shown to have a high binding affinity to MC receptors. Furthermore, the compounds of the invention influence the activity of one or more MC receptors, i.e. either by stimulating the activity of one or more MC-receptors or by inhibiting the activity of one or more MC receptors. Thus, the compounds of the present invention are either agonists or antagonists of a specific MC-receptor e.g. the MC-1, MC-2, MC-3, MC-4 or MC-5 receptor or agonists or antagonists of two or more MC-receptors (e.g. the MC-1, MC-2, MC-3, MC-4 and/or MC-5 receptors).

The MC-receptors belong to the class of G-protein coupled receptors which are all built from a single polypeptide forming 7 transmembrane domains. Five such receptors types, termed MC-1, MC-2, MC-3, MC-4 and MC-5, have been described. The MC receptor's signalling is mainly mediated via cAMP, but other signal transduction pathways are also known. The MC receptors are distinctly distributed in the body. The endogenous ligands for the MC receptors are the melanocortins, which are a group of pituitary peptide hormones that include adrenocorticotropin (ACTH) and the alpha, beta and gamma melanocyte-stimulating hormones (MSH) that derive from the prohormone proopiomelanocortin.

The MC-2 receptor is known as the adrenocorticotrophic hormone receptor since it selectively binds ACTH. The other melanocortin receptors are semi-selective in their ability to bind multiple melanocortins to some degree.

MC receptors are linked to a variety of physiological actions that are thought to be mediated by distinct subtypes of the MC receptors. In many cases, however, it is not entirely clear which of the subtypes is responsible for the effect as exemplified by the finding that selective MC-1 receptor agonists have a marked anti-inflammatory action, but seem to lack the organ protective effect described for unspecific MC receptor agonists as α-MSH, where it has been suggested that additional MC-3 and/or MC-5 receptor stimulation is needed to get the organ protective effect. Another example is the central effects of melanocortin receptor stimulation where it is unclear whether stimulation of both the MC-3 and MC-4 receptor is needed or whether only stimulation of one of these receptors is adequate to obtain central effects.

It has long been known that MSH-peptides may affect many different processes such as motivation, learning, memory, behaviour (including feeding and sexual behaviour), inflammation (including immunostimulatory and immunosuppressive), body temperature, pain perception, blood pressure, heart rate, vascular tone, brain blood flow, trophic effects in different organs, nerve growth, placental development, endocrine and exocrine functions, aldosterone synthesis and release, thyroxin release, spermatogenesis, ovarian weight, prolactin and FSH secretion, effects or other hormones, uterine bleeding in women, sebum and pheromone secretion, blood glucose levels, natriuresis, intrauterine foetal growth, as well as other events surrounding parturition, (see, for example, Eberle: The melanotropins: Chemistry, physiology and mechanisms of action. Basel: Karger, Switzerland. 1988, ISBN 3-8055-4678-5; Gruber et al., Am. J. Physiol. 1989, 257, R681-R694; De Wildt et al., J. Cardiovascular Pharmacology. 1995, 25, 898-905) as well as inducing natriuresis (Tin et al., Hypertension. 1987, 10, 619-627).

Moreover, it is also well-known that the immunomodulatory action of α-MSH includes both immunostimulatory and immunosuppressive effects. Several studies have shown that α-MSH antagonises the effects of pro-inflammatory cytokines such as IL-1α, IL-1β, IL-6 and TNF-α, and induces the production of the antiinflammatory cytokine, IL-10 (for review, see Catania & Lipton, Endocr Rev. 1993 Oct;14(5):564-76).

Eating behaviour is regulated by a complex network of physiological regulatory pathways that involve both the central nervous system and peripheral sites. Factors such as leptin, insulin, NPY (neuropeptide Y), orexins, CRF (Corticotropin-Releasing Factor, release hormone) and melanocortic peptides (Schwartz, Nature Medicine 1998, 4, 385-386) are known to control the amount of food intake, which may affect body weight, body fat mass and growth rate. Recent studies have shown a role of MC-receptors, especially the MC-4 receptor, for control of food intake, and there is evidence indicating that the melanocortins and the MC4 receptor are important factors downstream of leptin. Intracerebroventricular injections of the melanocortic peptides α-MSH and ACTH(1-24) have been shown to markedly inhibit feeding (Poggioli et al., Peptides, 1986, 7, 843-848; Vergoni et al., Neuropeptides, 1986, 7, 153-158).

The MC-5 receptor has recently been attributed a role in control of exocrine gland function 5 (van der Kraan, et al., Endocrinol. 1998, 139, 2348-2355; Chen et al., Cell. 1997, 91, 789-798).

In addition, the melanocortic peptides have distinct effects on sexual functions in that they cause erection in males (Donovan, Psychol. Med., 1978, 8, 305-316), presumably mediated by a central agonistic effect of the peptide on MC receptors. It has also been shown that a MC receptor blocker could inhibit the erectogenic effect of melanocortic peptides (Vergoni et al., Eur. J. Pharmacol., 1998, 362; 95-101).

The compounds of the present invention have valuable therapeutic properties, making them useful for the treatment of inflammatory conditions and/or diseases, as exemplified below. Thus, in one embodiment, the invention relates to the use of a compound of formula (I) for the manufacture of a medicament for the treatment of a disease selected from the group consisting of inflammatory conditions, e.g. acute or chronic inflammatory conditions, such as arthritis, including diseases associated with arthritis, osteoartritis, rheumatoid arthritis, spondylarthropathies (e.g. ankylosing spondilitis), reactive arthritis (including arthritis following rheumatic fever), Henoch-Schonlein purpura, Reiter's disease, uveitis and connective tissue disorders such as systemic lupus erythematosus, polymyositis/dermatomyositis, systemic sclerosis, mixed connetive tissue disease, sarcoidosis and primary Sjogrens syndrome including keratoconjunctivitis sicca, polymyalgia rheumatica, and other types of vasculitis, crystal deposition diseases (including gout), pyrophosphate arthropathy, acute calcific periarthritis; inflammatory bowel disease (including Chron's disease and ulcertive colitis), diverticular disease of the colon, and irritable bowel syndrome, pancreatitis, inflammatory upper and lower airway diseases such as chronic obstructive pulmonary diseases (COPD), allergic and non-allergic asthma, allergic rhinitis, allergic and non-allergic conjunctivitis, allergic and non-allergic dermatitis, atopic dermatitis, trauma and post operative stress syndromes, diabetes mellitus, diabetes mellitus type I, diabetes mellitus type II, obesity-induced diabetes mellitus, insulin-resistance, metabolic syndrome, sexual dysfunction including dysfunction of male erection, eating disorders including anorexia, obesity, mental disorders, dysfunction of the endocrine system, drug-induced disorders of the blood and lymphoid system, allergy disorders, disorders of the cardiovascular system and pain.

Furthermore, the invention relates to a method of treating a mammal having a disease or disorder selected from the group consisting of inflammatory conditions, e.g. acute or chronic inflammatory conditions, such as arthritis, including diseases associated with arthritis, osteoartritis, rheumatoid arthritis, spondylarthropathies (e.g. ankylosing spondilitis), reactive arthritis (including arthritis following rheumatic fever), Henoch-Schonlein purpura, Reiter's disease, uveitis and connective tissue disorders such as systemic lupus erythematosus, polymyositis/dermatomyositis, systemic sclerosis, mixed connetive tissue disease, sarcoidosis and primary Sjogrens syndrome including keratoconjunctivitis sicca, polymyalgia rheumatica, and other types of vasculitis, crystal deposition diseases (including gout), pyrophosphate arthropathy, acute calcific periarthritis; inflammatory bowel disease (including Chron's disease and ulcertive colitis), diverticular disease of the colon, and irritable bowel syndrome, pancreatitis, inflammatory upper and lower airway diseases such as chronic obstructive pulmonary diseases (COPD), allergic and non-allergic asthma, allergic rhinitis, allergic and non-allergic conjunctivitis, allergic and non-allergic dermatitis, atopic dermatitis, trauma and post operative stress syndromes, diabetes mellitus, diabetes mellitus type I, diabetes mellitus type II, obesity-induced diabetes mellitus, insulin-resistance, metabolic syndrome, sexual dysfunction including dysfunction of male erection, eating disorders including anorexia, obesity, mental disorders, dysfunction of the endocrine system, drug-induced disorders of the blood and lymphoid system, allergy disorders, disorders of the cardiovascular system and pain, said method comprising administering to said mammal a therapeutically effective amount of a compound of the invention. The compound of the invention is defined by formula (I).

In the following the conditions and diseases of which the compounds of the present invention are useful for treating and/or alleviating and/or partially or significantly preventing, are described in details.

### Inflammatory conditions

Compounds of formula (I) and/or their pharmaceutically acceptable salts have valuable pharmacological properties, making them useful for the treatment of inflammation, an inflammatory condition or an inflammatory disease such as inflammation related to the production of nitric oxide, inflammation related to increased amounts (upregulated amounts) of inducible nitric oxide synthase, inflammation related to activation of transcriptional activators, inflammation related to nuclear factor kappa beta, inflammation related to macrophages, neutrophils, monocytes, keratinocytes, fibroblasts, melanocytes, pigment cells and endothelial cells, inflammation related to increased production and/or release of inflammatory cytokines, such as e.g. interleukins, in particular interleukin 1 (IL-1), interleukin 6 (IL-6) and tumor necrosis factor a (TNF-α).

In the present specification, "increased production" refers to increased formation, increased release, or increased amount of an endogenous compound locally, regionally or systemically in a patient compared to the amount of said endogenous compound in a healthy individual. In the present specification, "upregulated" refers to an increased activity or amount of the compound compared with that in a healthy individual.

In the present specification "decreased production" refers to decreased formation, decreased release, or decreased amount of an endogenous compound in a patient compared to the amount of said endogenous compound in a healthy individual. In the present specification "downregulated" refers to a decreased activity or amount of the compound compared with that in a healthy individual.

In particular, positive treatment effects or preventive effects may be seen in conditions
where inflammation or an inflammatory-like condition is caused by or being associated with one or more of the following: allergy, hypersensitivity, bacterial infection, viral infection, inflammation caused by toxic agent, fever, autoimmune disease, radiation damage by any source including UV-radiation, X-ray radiation, γ-radiation, α- or β-particles, sun burns, elevated temperature or mechanical injury. Moreover, inflammation due to hypoxia, which is optionally followed by reoxygenation of the hypoxic area, is typically followed by severe inflammation, which condition may be positively affected by treatment with a compound of the invention.

In very specific embodiments of the invention, a compound of the invention may be administered for the prevention or therapeutic treatment of inflammatory diseases of the skin (including the dermis and epidermis) of any origin, including skin diseases having an inflammatory component. Specific examples of this embodiment of the invention include treatment of contact dermatitis of the skin, atopic dermatitis, sunburns of the skin, burns of any cause, and inflammation of the skin caused by chemical agents, psoriasis, vasculitis, pyoderma gangrenosum, discoid lupus erythematosus, eczema, pustulosis palmo-plantaris, and phemphigus vulgaris.

Moreover inflammatory diseases include all kinds of soft-tissue rheumatism including rheumatoid arthritis, bursitis, tenosynovitis or peritendonitis, enthesitis, nerve compression, periarthritis or capsulitis, muscle tension and muscle dysfunction. Furthermore, inflammatory diseases include all kinds of arthritis in children such as Juvenile Chronic arthritis including Still's disease, juvenile rheumatoid arthritis, juvenile ankylosing spondylitis.

Also comprised by the invention is the administration of a compound of formula (I) or a pharmacologically acceptable salt thereof for the treatment of an inflammatory disease in the abdomen, including an abdominal disease having an inflammatory component. Specific examples of the treatment of such a disease with a compound of the invention are gastritis, including one of unknown origin, gastritis perniciosa (atrophic gastritis), ulcerous colitis (colitis ulcerosa), morbus Crohn (Chrons disease), systemic sclerosis, ulcus duodeni, coeliac disease, oesophagitis, ulcus ventriculi, acute and chronic gastritis, helicobacteer pylori infection, coeliac disease, gluten sensitive enteropathy, dermatitis herpitiformis, tropical sprue, Whipple's diease, radiation enteritis, systemic amyloidosis, eosinophilic gastroenteritis, intestinal lympangiectasia, inflammatory bowel disease, diverticular disease of the colon, and irritable bowel syndrome.

Comprised by the invention is also the administration of a compound of formula (I) or a pharmacologically acceptable salt thereof for the treatment of systemic or general and/or local immunological diseases, including those of an autoimmune nature, and other inflammatory diseases of a general nature. Specific examples include treatment of rheumatoid arthritis, psoriatic arthritis, systemic sclerosis, polymyalgia rheumatica, Wegener's granulomatosis, sarcoidosis, eosinophilic fasceitis, reactive arthritis, Bechterew's disease, systemic lupus erythematosus, arteritis temporalis, Behcet's disease, morbus Burger, Good Pastures' syndrome, eosinophilic granuloma, fibromyalgia, myositis, and mixed connective tissue disease. Included therein is also arthritis, including arthritis of unknown origin.

Further included in the invention is administration of a compound of formula (I) or a pharmacologically acceptable salt thereof for the treatment of a disease of the peripheral and/or central nervous system related to inflammation. Included in this aspect of the invention is the treatment of cerebral vasculitis, multiple sclerosis, autoimmune ophthalmitis and polyneuropathia. Comprised by the invention is also the administration of a compound of the invention for the treatment of an inflammation of the central nervous system to prevent apoptotic cell death. Moreover, as some of the compounds of the invention show a distinct ability to induce nerve regeneration, positive treatment effects are often seen in central nervous system diseases involving damage of cells in this region.

This aspect of the invention also includes treatment of traumatic injuries to the central nervous system, brain edema, multiple sclerosis, Alzheimer's disease, bacterial and viral infections in the central nervous system, stroke, and haemorrhagia in the central nervous system.

Comprised by the invention is also the administration of a compound of formula (I) or a pharmacologically acceptable salt thereof for the treatment of diseases of the eye and tear glands related to inflammation. Specific examples of such diseases comprise uveitis including anterior and posterior uveitis, retinal vasculitis, optic neuritis, optic neuromyelitis, Wegener's granulomatosis, Sjögren's syndrome, episcleritis, scleritis, sarcoidosis affecting the eye and polychondritis affecting the eye.

Comprised by the invention is also the administration of a compound of formula (I) or a pharmacologically acceptable salt thereof for the treatment of diseases of the ear related to inflammation, specific examples of which include polychondritis affecting the ear and external otitis.

Comprised by the invention is also the administration of a compound of formula (I) or a pharmacologically acceptable salt thereof for the treatment of diseases of the nose related to inflammation, specific examples of which are sarcoidosis, polychondritis and mid-line granuloma of the nose.

Comprised by the invention is also the administration of a compound of formula (I) or a pharmacologically acceptable salt thereof for the treatment of diseases related to inflammation of the mouth, pharynx and salivary glands. Specific examples include Wegener's granulomatosis, mid-line granuloma, Sjögren's syndrome and polychondritis in these areas.

Included in the invention is also the administration of a compound of formula (I) or a pharmacologically acceptable salt thereof for the treatment of diseases related to inflammation in the lung and/or airways, such as e.g. acute or chronic or subchronic inflammation in the lung and/or airway. Specific examples include treatment of idiopathic alveolitis, primary pulmonary hypertension, bronchitis, chronic bronchitis, sarcoidosis, alveolitis in inflammatory systemic disease, pulmonary hypertension in inflammatory systemic disease, Wegener's granulomatosis, Good Pastures' syndrome, upper and lower airway diseases such as chronic obstructive pulmonary disease (COPD), exacerbations in COPD, allergic and non-allergic asthma, allergic rhinitis, allergic and non-allergic conjunctivitis, acute respiratory diseases and/or chronic and/or subchronic airway and lung diseases.

Comprised by the invention is also the administration of a compound of formula (I) or a pharmacologically acceptable salt thereof for the treatment of diseases related to the inflammation of the heart. Specific examples include treatment of pericarditis, idiopathic pericarditis, myocarditis, Takayasus' arteritis, Kawasaki's disease, coronary artery vasculitis, pericarditis in inflammatory systemic disease, myocarditis in inflammatory systemic disease, endocarditis and endocarditis in inflammatory systemic disease.

Comprised by the invention is also the administration of a compound of formula (I) or a pharmacologically acceptable salt thereof for the treatment of diseases related to inflammation of the liver. Specific examples include treatment of hepatitis, chronic active hepatitis, biliary cirrhosis, hepatic damage by toxic agents, interferon induced hepatitis, hepatitis induced by viral infection, liver damage induced by anoxia and liver damage caused by mechanical trauma.

Comprised by the invention is also the administration of a compound of formula (I) or a pharmacologically acceptable salt thereof for the treatment of diseases related to inflammation of the pancreas. Specific examples include treatment (and prevention) of acute pancreatitis, chronic pancreatitis.

Moreover, comprised by the invention is also the administration of a compound of formula (I) or a pharmacologically acceptable salt thereof for the treatment of diseases related to conditions with increased fasting levels of LDL-Cholesterol, conditions with combined increased fasting levels of LDL-Cholesterol and triglyceride, conditions with increased fasting levels of triglyceride and conditions with increased fasting levels of HDL-Cholesterol.

Comprised by the invention is also the administration of a compound of formula (I) or a pharmacologically acceptable salt thereof for the treatment of diseases related to the inflammation of the thyroidea. Specific examples of these embodiments of the invention include treatment of thyreoiditis, autoimmune thyreoiditis and Hashimoto's thyreoiditis.

Comprised by the invention is also the administration of a compound of formula (I) or a pharmacologically acceptable salt thereof for the treatment of diseases related to inflammation of the kidney. Specific examples include treatment of glomerulonephritis, glomerulonephritis in systemic lupus erythematosus, periarteritis nodosa, Wegener's granulomatosis, Good-Pastures' syndrome, HLAb27 associated diseases, IgA nephritis (IgA = Immunoglobulin A), pyelonephritis, chronic pyelonephritis and interstitial nephritis.

Comprised by the invention is also the administration of a compound of formula (I) or a pharmacologically acceptable salt thereof for the treatment of diseases related to the inflammation of the joints. Specific examples include treatment of Bechterew's disease, psoriatic arthritis, rheumatoid arthritis, arthritis in colitis ulcerosa, arthritis in morbus Crohn, affection of joints in systemic lupus erythematosus, systemic sclerosis, mixed connective tissue disease, reactive arthritis, Reiter's syndrome. Moreover, included in this embodiment of the invention is treatment of arthrosis of any joint, in particular arthrosis of finger joints, the knee and the hip.

Comprised by the invention is also the administration of a compound of formula (I) or a pharmacologically acceptable salt thereof for the treatment of diseases related to the inflammation of blood vessels. Specific examples include treatment of arteritis temporalis, periarteritis nodosa, arteriosclerosis, Takayasus' arteritis and Kawasaki's disease. Particularly advantageous is the capacity of some compounds of the invention to afford protection against and prevention of arteriosclerosis. This is in part due to the capacity of some compounds of formula (I) or the pharmacologically acceptable salts thereof to prevent the induction of inducible nitric oxide synthesis (iNOS) caused by the action of oxidized Low Density Lipoprotein on endothelial cells and blood vessel walls.

Inflammatory diseases also include all kind of inflammatory conditions causing backpain including infections, septic discitis, tuberculosis, malignancies (such as matastases, myeloma and others), spinal tumours, ancylosing spondylitis, acute disc prolapse, chronic disc diseaase/osteoarthritis, osteoporosis, and osteomalacia. It also includes Pagets disease, hyperparathyroidism, renal osteodystrophy, spondylolisthesis, spinal senosis congenital abnormalities and fibromyalgia.

Comprised by the invention is also the administration of a compound of formula (I) or a pharmacologically acceptable salt thereof for the treatment of inflammation related to infections of any origin. Specific examples include treatment of inflammation secondary to infection caused by virus, bacteria, helminths, protozoae and fungus and include conditions such as AIDS, bacterial septicemia, systemic fungal infections, Rickettsial diseases, toxic shock syndrome, infectious mononucleosis, chlamydia thrachomatis, chlamydia psittaci, cytomegalovirus infection, campylobacter, salmonella, influenza, poliomyelitis, toxoplasmosis, Lassa Fever, Yellow Fever, billharziose, colibacteria, enterococcus, preteus, klebsiella, pseudomonas, staphylococcus aureus, staphylococcus epidermidis, candida albicans, tuberculosis, mumps, infectious mononucleosis, hepatitis and Coxackie virus.

Comprised by the invention is also the administration of a compound of formula (I) or a pharmacologically acceptable salt thereof for the treatment of inflammations related to trauma and/or tissue injury of any origin, such as e.g. a chemical trauma involving one or more toxic substances and/or drugs. Such drugs include tricyclic antidepressants, lithium salts, prenylamine, phenothizine derivatives, chemopreventive drugs including adriamycin. Also physical traumas including electromagnetic radiation may cause damages.

The invention relates to the use of a compound of formula (I) or a pharmacologically acceptable salt thereof for the manufacture of a medicament for the treatment and/or alleviation and/or partial or significant prevention of an inflammatory condition or an inflammatory disease as defined above.

Furthermore, the invention relates to a method of treating and/or alleviating and/or partially or significantly preventing an inflammatory condition or an inflammatory disease as defined above in a mammal, said method comprising administering to said mammal a therapeutically effective amount of a compound of formula (I) or a pharmacologically acceptable salt thereof.

### Insulin resistance and Diabetes mellitus

Comprised by the invention is also the administration of a compound of formula (I) or a pharmacologically acceptable salt thereof for the treatment of inflammations related to insulin resistance, metabolic syndrome, diabetes mellitus, including Type II diabetes mellitus where low grade inflammation in fatty tissue and muscles plays a significant role for the development of impairment in the signal transduction of insulin and thereby the development of insulin resistance and eventually diabetes mellitus. Comprised by the invention is also the administration of a compound of formula (I) or a pharmacologically acceptable salt thereof for the treatment of insulin resistance, metabolic syndrome, diabetes mellitus, including Type II diabetes mellitus.

Thus, in one embodiment the invention relates to the use of a compound of formula (I) or a pharmacologically acceptable salt thereof for the manufacture of a medicament for the treatment and/or alleviation and/or partial or significant prevention of a disease or condition selected from the group consisting of insulin resistance, metabolic syndrome, diabetes mellitus, Type II diabetes mellitus and obesity-induced diabetes mellitus.

Furthermore, the invention also relates to a method of treating and/or alleviating and/or partially or significantly preventing a disease or condition selected from the group consisting of insulin resistance, metabolic syndrome, diabetes mellitus, Type II diabetes mellitus and obesity-induced diabetes mellitus in a mammal, said method comprising administering to said mammal a therapeutically effective amount of a compound of formula (I) or a pharmacologically acceptable salt thereof.

### Eating disorders

In another embodiment, the invention relates to the use of a compound of formula (I) or a pharmacologically acceptable salt thereof for the manufacture of a medicament for the treatment and/or alleviation and/or partial or significant prevention of eating disorders, such as e.g. anorexia and bulimia and inflammations related to eating disorders, such as e.g. anorexia and bulimia.

The invention also relates to a method of treating and/or alleviating and/or partially or significantly preventing eating disorders, such as e.g. anorexia and bulimia and inflammations related to eating disorders, such as e.g. anorexia and bulimia, said method comprising administering to said mammal a therapeutically effective amount of a compound of formula (I) or a pharmacologically acceptable salt thereof.

### Obesity

In a further embodiment, the invention relates to the use of a compound of formula (I) or a pharmacologically acceptable salt thereof for the manufacture of a medicament for the treatment and/or alleviation and/or partial or significant prevention of inflammation related to obesity where low grade inflammation in fatty tissue and muscles plays a significant role for the development of the complications to obesity. This includes the development of insulin resistance and eventually diabetes mellitus, e.g. diabetes mellitus type II, dyslipidemia, hypertension and aterosclerosis. The invention relates to the use of a compound of formula (I) or a pharmacologically acceptable salt thereof for the manufacture of a medicament for the treatment and/or alleviation and/or partial or significant prevention of a disease or condition selected from the group consisting of obesity, metabolic syndrome and obesity-induced diabetes mellitus, such as obesity-induced type II diabetes mellitus.

Furthermore, the invention also relates to a method of treating and/or alleviating and/or partially or significantly preventing inflammation related to obesity where low grade inflammation in fatty tissue and muscles plays a significant role for the development of the complications to obesity, insulin resistance, diabetes mellitus, e.g. diabetes mellitus type II, dyslipidemia, hypertension and aterosclerosis, said method comprising administering to said mammal a therapeutically effective amount of a compound of formula (I) or a pharmacologically acceptable salt thereof.

The invention relates to a method of treating and/or alleviating and/or partially or significantly preventing metabolic syndrome and/or obesity-induced diabetes mellitus, such as obesity-induced type II diabetes mellitus, said method comprising administering to said mammal a therapeutically effective amount of a compound of formula (I) or a pharmacologically acceptable salt thereof.

### Congestive heart failure

In yet a further embodiment, the invention relates to the use of a compound of formula (I) or a pharmacologically acceptable salt thereof for the manufacture of a medicament for the treatment and/or alleviation and/or partial or significant prevention of inflammation related to congestive heart failure where low grade inflammation including TNF-α production within the heart plays a significant role for the development of fibrosis and myocardial remodelling in the falling heart. The invention also relates to the use of a compound of formula (I) or a pharmacologically acceptable salt thereof for the manufacture of a medicament for the treatment and/or alleviation and/or partial or significant prevention of congestive heart failure.

Furthermore, the invention relates to a method of treating and/or alleviating and/or partially or significantly preventing inflammations related to congestive heart failure where low grade inflammation including TNF-α production within the heart plays a significant role for the development of fibrosis and myocardial remodelling in the falling heart, said method comprising administering to said mammal a therapeutically effective amount of a compound of formula (I) or a pharmacologically acceptable salt thereof. Furthermore, the invention relates to a method of treating and/or alleviating and/or partially or significantly preventing congestive heart failure, said method comprising administering to said mammal a therapeutically effective amount of a compound of formula (I) or a pharmacologically acceptable salt thereof.

### Sexual dysfunction

In still a further embodiment, the invention relates to the use of a compound of formula (I) or a pharmacologically acceptable salt thereof for the manufacture of a medicament for the treatment and/or alleviation and/or partial or significant prevention of sexual functions and/or dysfunctions such as inducing erection in man, to induce erection in animal breeding, to stimulate intercourse in animals which are difficult to mate, in particular rare species or valuable strains, pets, cats, dogs, horses or to reduce sexual behaviour in animals, e.g. for pets, cats etc., impotence and disorders related to sexual drive, including lack of sexual drive or abnormal sexual drive in both men and women.

Moreover, the invention relates to a method of treating and/or alleviating and/or partially or significantly preventing sexual functions and/or dysfunctions such as inducing erection in man, to induce erection in animal breeding, to stimulate intercourse in animals which are difficult to mate, in particular rare species or valuable strains, pets, cats, dogs, horses or to reduce sexual behaviour in animals, e.g. for pets, cats etc., impotence and disorders related to sexual drive, including lack of sexual drive or abnormal sexual drive in both men and women, said method comprising administering to said mammal a therapeutically effective amount of a compound of formula (I) or a pharmacologically acceptable salt thereof.

### Mental disorders

The invention also relates to the use of a compound of formula (I) or a pharmacologically acceptable salt thereof for the manufacture of a medicament for the treatment and/or alleviation and/or partial or significant prevention of mental disorders such as psychoses, depression, anxiety, senile dementia, Alzheimer's disease, drug abuse disorders and eating disorders such as anorexia and bulimia.

Furthermore, the invention relates to a method of treating and/or alleviating and/or partially or significantly preventing mental disorders such as psychoses, depression, anxiety, senile dementia, Alzheimer's disease, drug abuse disorders and eating disorders such as anorexia and bulimia, said method comprising administering to said mammal a therapeutically effective amount of a compound of formula (I) or a pharmacologically acceptable salt thereof.

### Dysfunction of the endocrine system

In still another embodiment, the invention relates to the use of a compound of formula (I) or a pharmacologically acceptable salt thereof for the manufacture of a medicament for the treatment and/or alleviation and/or partial or significant prevention of dysfunctions of the endocrine system and other hormonal systems such as excessive menstruations, endometriosis, events related to parturition, dysfunctions related to prolactin, dysfunctions related to growth hormone, dysfunctions related to testosterone, dysfunctions related to estrogen, dysfunctions related to glucocorticoids, dysfunctions related to luteinizing hormone and follicle stimulating hormone, inducing abortion, for prevention of abortion and/or for treatment of events related to parturition.

The invention also relates to a method of treating and/or alleviating and/or partially or significantly preventing dysfunctions of the endocrine system and other hormonal systems such as excessive menstruations, endometriosis, events related to parturition, dysfunctions related to prolactin, dysfunctions related to growth hormone, dysfunctions related to testosterone, dysfunctions related to estrogen, dysfunctions related to glucocorticoids, dysfunctions related to luteinizing hormone and follicle stimulating hormone, abortion, events related to parturition, said method comprising administering to said mammal a therapeutically effective amount of a compound of formula (I) or a pharmacologically acceptable salt thereof.

### Drug-induced disorders of the blood and lymphoid system

In still another embodiment, the invention relates to the use of a compound of formula (I) or a pharmacologically acceptable salt thereof for the manufacture of a medicament for the treatment and/or alleviation and/or partial or significant prevention of drug-induced disorders of the blood and lymphoid system, including the treatment of drug-induced hypersensitivity (including drug hypersensitivity) affecting blood cells and blood cell forming organs (e.g. bone marrow and lymphoid tissue). Specific embodiments of this aspect of the invention include the treatment of anemia, granulocytopenia, thrombocytopenia, leukopenia, aplastic anemia, autoimmune hemolytic anemia, autoimmune thrombocytopenia and autoimmune granulocytopenia.

The invention also relates to a method of treating and/or alleviating and/or partially or significantly preventing drug-induced disorders of the blood and lymphoid system, including the treatment of drug-induced hypersensitivity (including drug hypersensitivity) affecting blood cells and blood cell forming organs (e.g. bone marrow and lymphoid tissue), said method comprising administering to said mammal a therapeutically effective amount of a compound of formula (I) or a pharmacologically acceptable salt thereof.

### Allergy disorders

In yet a further embodiment, the invention relates to the use of a compound of formula (I) or a pharmacologically acceptable salt thereof for the manufacture of a medicament for the treatment and/or alleviation and/or partial or significant prevention of fast allergic disorders (Type I allergy).

Moreover, the invention relates to a method of treating and/or alleviating and/or partially or significantly preventing drug-induced disorders of the blood and lymphoid system, including the treatment of fast allergic disorders (Type I allergy), said method comprising administering to said mammal a therapeutically effective amount of a compound of formula (I) or a pharmacologically acceptable salt thereof.

Examples of fast allergic disorders are anaphylactic reactions, anaphylactoid reactions, asthma, asthma of allergic type, asthma of unknown origin, rhinitis, hay fever and pollen allergy.

### Disorders of the cardiovascular system

In one embodiment, the invention relates to the use of a compound of formula (I) or a pharmacologically acceptable salt thereof for the manufacture of a medicament for the treatment and/or alleviation and/or partial or significant prevention of disorders of the cardiovascular system such as disorders related to blood pressure, heart rate, vascular tone, natriuresis, bleeding, shock, disorders related to ischemia, infarction, reperfusion injuries, arrhythmias of the heart, in particular during ischemia, or for the treatment of arrhythmias associated with reoxygenation of a previously ischemic period of the heart.

The invention also relates to a method of treating and/or alleviating and/or partially or significantly preventing disorders of the cardiovascular system such as disorders related to blood pressure, heart rate, vascular tone, natriuresis, bleeding, shock, disorders related to ischemia, infarction, reperfusion injuries, arrhythmias of the heart, in particular during ischemia, or arrhythmias associated with reoxygenation of a previously ischemic period of the heart, said method comprising administering to said mammal a therapeutically effective amount of a compound of formula (I) or a pharmacologically acceptable salt thereof.

### Pain

In another embodiment, the invention relates to the use of a compound of formula (I) or a pharmacologically acceptable salt thereof for the manufacture of a medicament for the treatment and/or alleviation and/or partial or significant prevention of pain such as pain of central origin, pain seen after damage to the CNS, stroke, infarction, pain of peripheral origin, chronic pain, neuropathies and disorders where a treatment effect is achieved by stimulation of receptors in the periaqueductal grey area.

Moreover, the invention relates to a method of treating and/or alleviating and/or partially or significantly preventing pain such as pain of central origin, pain seen after damage to the CNS, stroke, infarction, pain of peripheral origin, chronic pain, neuropathies and disorders where a treatment effect is achieved by stimulation of receptors in the periaqueductal grey area, said method comprising administering to said mammal a therapeutically effective amount of a compound of formula (I) or a pharmacologically acceptable salt thereof.

### Other uses

### Skin tanning

Because of the capacity of compounds of the invention to stimulate pigment formation in epidermal cells, some of the compounds of the invention may be also useful for inducing skin tanning for cosmetic reasons, for treatment of vitiligo, or any other condition where darkening of skin color is desired. Moreover, because of the ability of some of the compounds of the invention to inhibit pigment formation in cells of the skin, they may also be useful for inducing lighter skin color for cosmetic reasons, or during any condition
where a lighter color of skin is desired.

Compounds of formula (I) or the pharmaceutically acceptable salts thereof have valuable pharmacological properties, making them useful to cause skin tanning, darkening the colour of the skin, to induce melanin synthesis in the skin, to reduce skin tanning, lightening the colour of the skin, to reduce or block melanin synthesis in the skin, to cause anti-inflammatory actions in the skin, to modulate epidermal growth, to improve wound healing, to treat acne, seborrhoea, acne roseacea, atopic dermatitis, psoriasis and conditions related to malfunctions of the glands of the skin, e.g. sebacous glands and over or underproduction of sebum.

### In vivo formation of second messenger elements

Compounds of the invention are useful for inhibiting or stimulating the in vivo formation of second messenger elements such as cAMP. Such inhibition/stimulation may be used in cells or crushed cell systems in vitro, e.g. for analytical or diagnostic purposes.

### Labels and tags

For analytical and diagnostic purposes the compounds of the invention may be used in radioactive form where they comprise one or more radioactive labels or gamma or positron emitting isotopes, to be used in radioligand binding for the quantification as well as tissue localisation of MC-receptors, for analysis of dissociation/association constants, and for imaging of in vivo binding by the use of scintigraphy, positron emission tomography (PET) or single photon emission computed tomography (SPECT), or for the diagnosis of disease and treatment of any malignancy where the malignant cells contain MC receptors.

Alternatively the compounds of the invention can be labelled with any other type of label that allows detection of the respective compound, e.g. fluorescence, biotin, NMR, MRI, or labels activated by gamma-irradiation, light photons or biochemical processes, or by light or UV-light (the latter in order to obtain a compound useful for covalent labelling of MC receptors by a photoaffinity technique).

Compounds of formula (I) or the pharmacologically acceptable salts thereof may also be tagged with a toxic agent (i.e. doxorubicin, ricin, diphtheria toxin or other) and used for targeted delivery to malignant cells bearing MC receptors, or tagged with a compound capable of activating the endogenous immune system for triggering the immune system (for example a compound, monoclonal antibody or other, capable of binding to a T-cell antigen, e.g. CD3 or other) for treatment of malignancies and other MC receptor expressing diseases. The thus formed hybrid compound will direct cytotoxic cells to the malignant melanoma cells or the MC1-receptor bearing malignant cells and inhibit the tumor growth.

Compounds of formula (I) or a pharmacologically acceptable salt thereof may be attached to the antibody chemically by covalent or non-covalent bond(s).

Compounds of the invention may be used for the treatment and diagnosis of diseases, disorders and/or pathological conditions in an animal, in particular in man.

The compounds of the present invention may be bound covalently or non-covalently to one or several of other molecule(s) of any desired structure(s); the thus formed modified compound or complex may be used for the same purposes as described in this specification for the compounds of the invention, as well as is disclosed in the Examples given below. In a particularly important embodiment of the invention, a radioactively-labelled molecule is covalently bound to a compound of formula (I) or a pharmacologically acceptable salt thereof so as to make a compound of formula (I) or a pharmacologically acceptable salt thereof radioactively labelled.

Some of the compounds of the invention have an effect on xanthine oxidase in mammals, including humans.

The invention is further illustrated by the following non-limiting examples.

### EXPERIMENTAL

### Example 1 - Preparation of Compound 1

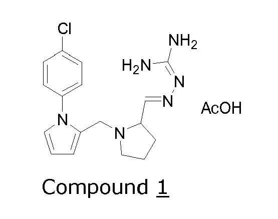

### Step A

1-4-(Chlorophenyl)-1H-pyrrole-2-carboxaldehyde (1 eqv.) and pyrrolidin-2-yl-methanol (2 eqv.) are dissolved in 2% AcOH in MeOH, and NaCNBH₃ (3 eqv.) is added. The mixture is stirred at room temperature for 18 h and subsequently evaporated and purified by flash chromatography to give the alcohol.

### Step B

The alcohol from step A (1 eqv.) is dissolved in DMSO:DCM (3:1). Triethylamine (5 eq.) and subsequently SO₃-pyridine complex (5 eqv.) are added and the reaction mixture is stirred at room temperature for 1 h. The reaction mixture is subsequently diluted with H₂O and extracted with EtOAc. The combined layers are washed with 1 N HCl and brine, dried (Na₂SO₄), filtered and evaporated *in vacuo* to give the desired aldehyde.

### Step C

The aldehyde from step B (1 eqv.) and aminoguanidine bicarbonate (1.1 eqv.) is mixed in THF and refluxed for 30 minutes. The solvent is the removed *in vacuo* and a mixture of acetonitrile and AcOH (2 eqv.) is added to the residue. The solvent is evaporated and the residue is re-crystallised.

### Example 2 - In vitro pharmacology and binding assays

### Description of applied methods

Determination of binding affinities for MC receptors is performed by [¹²⁵I]-[Nle4,D-Phe7]α-MSH ([¹²⁵I]-NDP-MSH) radio-ligand binding. In short, murine B16-F1 melanoma cells expressing MC1, but not other MC receptors, are used for binding affinity studies against the murine MC1 receptor (Siegrist et al.; 1988, J. Recept. Res., 8(1-4):323-43). For human MC3, MC4 and MC5 receptor affinities, human recombinant CHO cells are used (Schioth et al. 1997, Neuropeptides 31:565-71,1997). Cells are suspended in HEPES buffer and transferred to microwell plates. Radio-ligand, as well as test compound, in the concentration range of 10⁻¹⁰ to 10⁻⁶ are added to the cells. After incubation at 37°C (22°C for the MC1 receptor assay) separation of bound and free [¹²⁵I]-NDP-MSH is performed by multiple washings with buffer.

The results are expressed as a percent of control specific binding obtained in the presence of the test compounds. Mean values for each assay are presented in Table I below. The IC₅₀ values (concentration causing a half-maximal inhibition of control specific binding) and Hill coefficients (*n_{H}*) are determined by non-linear regression analysis of the competition curves using Hill equation curve fitting. The inhibition constants (Kᵢ) are calculated from the Cheng Prusoff equation (Kᵢ = IC₅₀/(1+(L/K_{D})), where L = concentration of radio-ligand in the assay, and K_{D} = affinity of the radio-ligand for the receptor).

**Table I**

| Assay | Ligand | Conc. | Non Specific | Incubation |
|---|---|---|---|---|
| MC₁ | [¹²⁵I]NDP-MSH | 0.05 nM | NDP-MSH | 90 min/22°C |
| | | | (1 µM) | |
| MC₃ *(h)* | [¹²⁵I]NDP-MSH | 0.075 nM | NDP-MSH | 60 min/37°C |
| | | | (1 µM) | |
| MC₄ *(h)* | [¹²⁵I]NDP-MSH | 0.05 nM | NDP-MSH | 120 min/37°C |
| | | | (1 µM) | |
| MC₅ *(h)* | [¹²⁵I]NDP-MSH | 0.05 nM | NDP-MSH | 60 min/37°C |
| | | | (1 µM) | |

## Claims

1. A compound of the general formula (I) 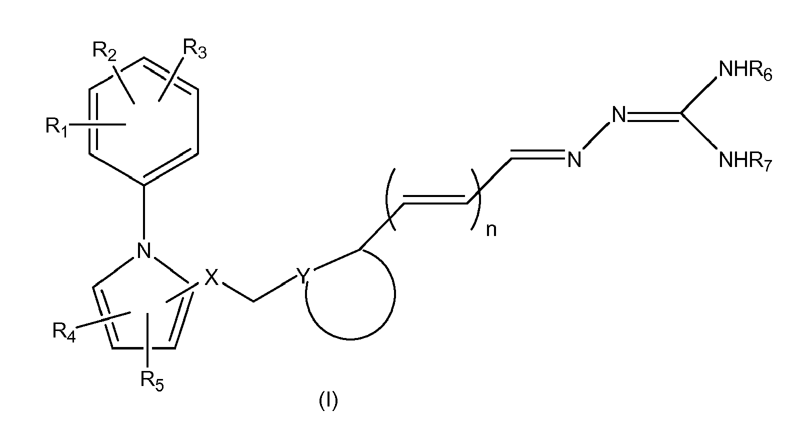 including tautomeric and isomeric forms, such as enantiomeric forms, diastereomeric forms and racemic forms, thereof,
wherein
X is (CH₂)ₘ, and m is 0, 1 or 2;
n is 0, 1 or 2;
Y is nitrogen (N) or carbon (C);
∪ represents, together with Y and the carbon atom covalently linked to Y, an optionally substituted five- or six-membered heterocyclyl or cycloalkyl group; each R₁, R₂, R₃, R₄ and R₅ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen,
where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine; each R₆ and R₇ is independently selected from the group consisting of hydrogen, optionally substituted C₁₋₆-alkyl, optionally substituted C₂₋₆-alkenyl, optionally substituted C₄₋₆-alkadienyl, optionally substituted C₂₋₆-alkynyl, optionally substituted C₁₋₆-alkoxycarbonyl, optionally substituted C₁₋₆-alkylcarbonyl, optionally substituted aryl, optionally substituted aryloxycarbonyl, optionally substituted arylcarbonyl, optionally substituted heteroaryl, optionally substituted heteroaryloxycarbonyl, optionally substituted heteroarylcarbonyl, aminocarbonyl, mono- and di(C₁₋₆-alkyl)aminocarbonyl, amino-C₁₋₆-alkyl-aminocarbonyl and mono- and di(C₁₋₆-alkyl)amino-C₁₋₆-alkyl-aminocarbonyl; or R₆ and R₇ may together form a five- or six-membered nitrogen-containing ring; or a pharmaceutically acceptable salt thereof.

2. The compound according to claim 1, wherein each R₁, R₂, R₃, R₄ and R₅ is independently selected from the group consisting of hydrogen, optionally substituted C₁₋₆-alkyl, optionally substituted C₂₋₆-alkenyl, optionally substituted C₂₋₆-alkynyl, hydroxy, optionally substituted C₁₋₆-alkoxy, optionally substituted C₂₋₆-alkenyloxy, carboxy, optionally substituted C₁₋₆ alkoxycarbonyl, optionally substituted C₁₋₆-alkylcarbonyl, formyl, amino, mono- and di(C₁₋₆-alkyl)amino, carbamoyl, mono- and di(C₁₋₆-alkyl)aminocarbonyl, amino-C₁₋₆-alkylaminocarbonyl, mono- and di(C₁₋₆-alkyl)amino-C₁₋₆-alkyl-aminocarbonyl, C₁₋₆-alkylcarbonylamino, amino-C₁₋₆-alkyl-carbonylamino, mono- and di(C₁₋₆-alkyl)amino-C₁₋₆-alkylcarbonylamino, cyano, carbamido, C₁₋₆-alkanoyloxy, C₁₋₆-alkylsulphonyl, C₁₋₆-alkylsulphinyl, C₁₋₆-alkylsulphonyloxy, aminosulfonyl, mono- and di(C₁₋₆-alkyl)aminosulfonyl, nitro, optionally substituted C₁₋₆-alkylthio and halogen.

3. The compound according to claim 2, wherein each R₁, R₂, R₃, R₄ and R₅ is independently selected from the group consisting of hydrogen, optionally substituted C₁₋₆-alkyl, optionally substituted C₂₋₆-alkenyl, hydroxy, optionally substituted C₁₋₆-alkoxy, amino, cyano, nitro and halogen.

4. The compound according to any of claims 1-3, wherein R₆ and R₇ are both hydrogen.

5. The compound according to any of claims 1-4, wherein R₄ is hydrogen and R₁, R₂, R₃ and R₅ are as defined in any of claims 1-3.

6. The compound according to claim 5, wherein R₁ and R₄ are hydrogen and R₂, R₃ and R₅ are as defined in any of claims 1-3.

7. The compound according to claim 6, wherein R₁, R₄ and R₅ are hydrogen and R₂ and R₃ are as defined in any of claims 1-3.

8. The compound according to claim 6 or 7, wherein R₂ is located in the 2-position and R₃ is located in the 3-position.

9. The compound according to claim 6 or 7, wherein R₂ is located in the 2-position and R₃ is located in the 4-position.

10. The compound according to claim 6 or 7, wherein R₂ is located in the 2-position and R₃ is located in the 5-position.

11. The compound according to claim 6 or 7, wherein R₂ is located in the 2-position and R₃ is located in the 6-position.

12. The compound according to claim 6 or 7, wherein R₂ is located in the 3-position and R₃ is located in the 4-position.

13. The compound according to claim 6 or 7, wherein R₂ is located in the 3-position and R₃ is located in the 5-position.

14. The compound according to claim 6 or 7, wherein R₂ is located in the 3-position and R₃ is located in the 6-position.

15. The compound according to claim 7, wherein R₁, R₂, R₄ and R₅ are hydrogen and R₃ is as defined in any of claims 1-3.

16. The compound according to claim 15, wherein R₃ is located in the 2-position.

17. The compound according to claim 15, wherein R₃ is located in the 3-position.

18. The compound according to claim 15, wherein R₃ is located in the 4-position.

19. The compound according to claim 15, wherein all of R₁, R₂, R₃, R₄ and R₅ are hydrogen.

20. The compound according to any of claims 1-19, wherein m is 0 or 1.

21. The compound according to claim 20, wherein m is 0.

22. The compound according to any of claims 1-21, wherein n is 0 or 1.

23. The compound according to claim 22, wherein n is 0.

24. The compound according to any of claims 1-23, wherein Y is carbon (C).

25. The compound according to claim 24, wherein ∪, together with Y and the carbon atom covalently linked to Y, represents an optionally substituted five- or six-membered cycloalkyl group.

26. The compound according to claim 25, wherein ∪, together with Y and the carbon atom covalently linked to Y, has the following chemical structure 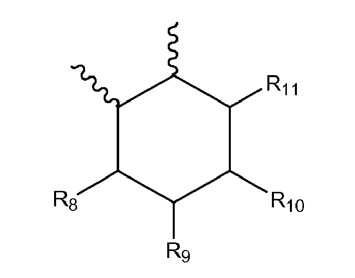 wherein each of R₈, R₉, R₁₀, and R₁₁ is independently selected from the group consisting of hydrogen, optionally substituted C₁₋₆-alkyl, optionally substituted C₃₋₆-cycloalkyl, optionally substituted C₂₋₆-alkenyl, optionally substituted C₄₋₆-alkadienyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, C₁₋₆-alkylsulphonylamino, optionally substituted aryl, optionally substituted aryloxycarbonyl, optionally substituted aryloxy, optionally substituted arylcarbonyl, optionally substituted arylamino, arylsulphonylamino, optionally substituted heteroaryl, optionally substituted heteroaryloxycarbonyl, optionally substituted heteroaryloxy, optionally substituted heteroarylcarbonyl, optionally substituted heteroarylamino, heteroarylsulphonylamino, optionally substituted heterocyclyl, optionally substituted heterocyclyloxycarbonyl, optionally substituted heterocyclyloxy, optionally substituted heterocyclylcarbonyl, optionally substituted heterocyclylamino, heterocyclylsulphonylamino, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkylcarbonylamino, mono- and di(C₁₋₆-alkyl)amino-C₁₋₆-alkyl-carbonylamino, cyano, guanidino, carbamido, C₁₋₆-alkanoyloxy, C₁₋₆-alkylsulphonyl, C₁₋₆-alkylsulphinyl, C₁₋₆-alkylsulphonyl-oxy, aminosulfonyl, mono- and di(C₁₋₆-alkyl)aminosulfonyl, nitro, optionally substituted C₁₋₆-alkylthio and halogen,
where any nitrogen-bound C₁₋₆-alkyl is optionally substituted with hydroxy, C₁₋₆-alkoxy, C₂₋₆-alkenyloxy, amino, mono- and di(C₁₋₆-alkyl)amino, carboxy, C₁₋₆-alkylcarbonylamino, halogen, C₁₋₆-alkylthio, C₁₋₆-alkyl-sulphonyl-amino or guanidine.

27. The compound according to claim 26, wherein each of R₈, R₉, R₁₀, and R₁₁ is independently selected from the group consisting of hydrogen, optionally substituted C₁₋₆-alkyl, optionally substituted C₂₋₆-alkenyl, optionally substituted C₂₋₆-alkynyl, hydroxy, optionally substituted C₁₋₆-alkoxy, optionally substituted C₂₋₆-alkenyloxy, carboxy, optionally substituted C₁₋₆-alkoxycarbonyl, optionally substituted C₁₋₆-alkylcarbonyl, formyl, amino, mono- and di(C₁₋₆-alkyl)amino, carbamoyl, mono- and di(C₁₋₆-alkyl)aminocarbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, amino-C₁₋₆-alkyl-carbonylamino, mono- and di(C₁₋₆-alkyl)amino-C₁₋₆-alkyl-carbonylamino, cyano, carbamido, C₁₋₆-alkanoyloxy, C₁₋₆-alkylsulphonyl, C₁₋₆-alkylsulphinyl, C₁₋₆-alkylsulphonyloxy, aminosulfonyl, mono- and di(C₁₋₆-alkyl)aminosulfonyl, nitro, optionally substituted C₁₋₆-alkylthio and halogen.

28. The compound according to claim 27, wherein each of R₈, R₉, R₁₀, and R₁₁ is independently selected from the group consisting of hydrogen, optionally substituted C₁₋₆-alkyl, optionally substituted C₂₋₆-alkenyl, hydroxy, optionally substituted C₁₋₆-alkoxy, amino, cyano, nitro and halogen.

29. The compound according to claim 28, wherein all of R₈, R₉, R₁₀, and R₁₁ are hydrogen.

30. The compound according to claim 25, wherein ∪, together with Y and the carbon atom covalently linked to Y, has the following chemical structure 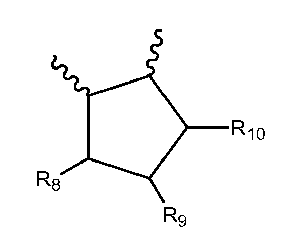 wherein each of R₈, R₉ and R₁₀ is as defined in any of claims 26-28.

31. The compound according to claim 30, wherein all of R₈, R₉ and R₁₀ are hydrogen.

32. The compound according to any of claims 1-23, wherein Y is nitrogen (N).

33. The compound according to claim 32, wherein ∪, together with Y and the carbon atom covalently linked to Y, represents an optionally substituted five- or six-membered heterocyclyl group.

34. The compound according to claim 33, wherein ∪, together with Y and the carbon atom covalently linked to Y, has the following chemical structure 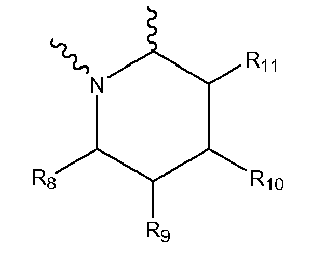 wherein each of R₈, R₉, R₁₀, and R₁₁ is as defined in any of claims 26-28.

35. The compound according to claim 34, wherein all of R₈, R₉, R₁₀ and R₁₁ are hydrogen.

36. The compound according to claim 33, wherein ∪, together with Y and the carbon atom covalently linked to Y, has the following chemical structure 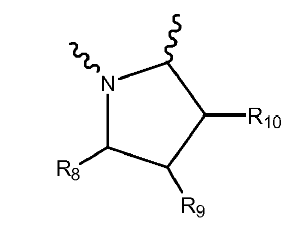 wherein each of R₈, R₉ and R₁₀ is as defined in any of claims 26-28.

37. The compound according to claim 36, wherein all of R₈, R₉ and R₁₀ are hydrogen.

38. The compound according to any of claims 1-37, wherein said compound has the structure shown in the general formula (Ia) 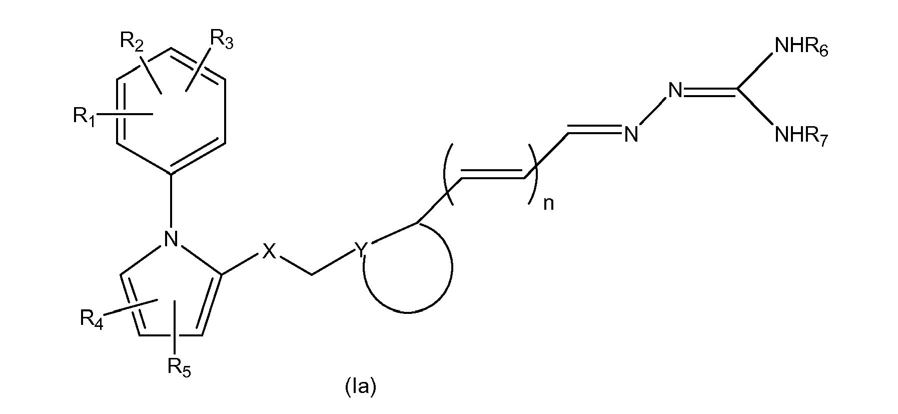 wherein R₁, R₂, R₃, R₄, R₅, R₆, R₇, X, Y and n are as defined in any of claims 1-37.

39. The compound according to any of claims 1-37, wherein said compound has the structure shown in the general formula (Ib) 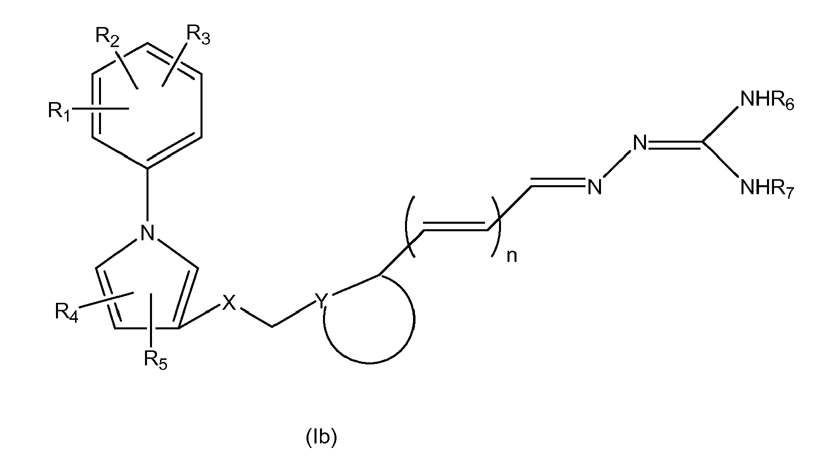 wherein R₁, R₂, R₃, R₄, R₅, R₆, R₇, X, Y and n are as defined in any of claims 1-37.

40. A pharmaceutical composition comprising a compound as defined in any of claims 1-39 and a pharmaceutically acceptable carrier or excipient.

41. A dosage form comprising the pharmaceutical composition as defined in claim 40.

42. The dosage form according to claim 41, wherein said dosage form is a solid dosage form.

43. The solid dosage form according to claim 42, wherein said solid dosage form is in the form of a tablet or capsule.

44. A compound as defined in any of claims 1-39 for use as a medicament.

45. Use of a compound as defined in any of claims 1-39 for the manufacture of a medicament for the treatment of obesity.

46. Use of a compound as defined in any of claims 1-39 for the manufacture of a medicament for the treatment of insulin-resistance.

47. Use of a compound as defined in any of claims 1-39 for the manufacture of a medicament for the treatment of diabetes mellitus.

48. Use of a compound as defined in any of claims 1-39 for the manufacture of a medicament for the treatment of obesity-induced diabetes mellitus.

49. Use of a compound as defined in any of claims 1-39 for the manufacture of a medicament for the treatment of rheumatoid arthritis.

50. Use of a compound as defined in any of claims 1-39 for the manufacture of a medicament for the treatment of atopic dermatitis.

51. Use of a compound as defined in any of claims 1-39 for the manufacture of a medicament for the treatment of intestinal bowel disease.
